(19) Europäisches Patentamt
European Patent Office
Office européen des brevets

(11) EP 1 349 822 B1

(12) EUROPÄISCHE PATENTSCHRIFT

(45) Veröffentlichungstag und Bekanntmachung des Hinweises auf die Patenterteilung:
**28.09.2016 Patentblatt 2016/39**

(21) Anmeldenummer: **02715410.3**

(22) Anmeldetag: **10.01.2002**

(51) Int Cl.:
***C07C 25/24*** (2006.01) ***C09K 19/34*** (2006.01)
***C09K 19/58*** (2006.01) ***C07C 25/22*** (2006.01)
***C07C 49/697*** (2006.01) ***C07D 307/91*** (2006.01)
***C09K 19/02*** (2006.01) ***C09K 19/32*** (2006.01)

(86) Internationale Anmeldenummer:
**PCT/EP2002/000191**

(87) Internationale Veröffentlichungsnummer:
**WO 2002/055463 (18.07.2002 Gazette 2002/29)**

# (54) FLUORIERTE AROMATEN UND IHRE VERWENDUNG IN FLÜSSIGKRISTALLMISCHUNGEN

FLUORINATED AROMATIC COMPOUNDS AND THE USE OF THE SAME IN LIQUID CRYSTAL MIXTURES

COMPOSES AROMATIQUES FLUORES ET LEUR UTILISATION DANS DES MELANGES DE CRISTAUX LIQUIDES

(84) Benannte Vertragsstaaten:
**DE FR GB**

(30) Priorität: **11.01.2001 DE 10101022**

(43) Veröffentlichungstag der Anmeldung:
**08.10.2003 Patentblatt 2003/41**

(73) Patentinhaber: **Merck Patent GmbH**
**64293 Darmstadt (DE)**

(72) Erfinder:
- **SCHMIDT, Wolfgang**
  **63303 Dreieich (DE)**
- **WINGEN, Rainer**
  **65795 Hattersheim (DE)**
- **HORNUNG, Barbara**
  **63594 Hasselroth (DE)**

(56) Entgegenhaltungen:
**EP-A- 0 325 035** **WO-A-01/10803**
**DE-A- 19 720 289**

- **DATABASE WPI Section Ch, Week 199846 Derwent Publications Ltd., London, GB; Class E19, AN 1998-537408 XP002192894 & JP 10 236992 A (CHISSO CORP), 8. September 1998 (1998-09-08) in der Anmeldung erwähnt**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 349 822 B1

Printed by Jouve, 75001 PARIS (FR)

## Beschreibung

Viele Hersteller haben elektrooptische Anzeigen auf der Basis nematischer Flüssigkristalle entwickelt, die seit einigen Jahren beispielsweise im Bereich von Notebook PCs, Personal Digital Assistants und Desktop Monitoren im Einsatz sind. Dabei werden die Technologien TN (Twisted Nematics), STN (Supertwisted Nematics), AM-TN (Active Matrix - Twisted Nematics), AM-IPS (Active Matrix - In Plane Switching), AM-MVA (Active Matrix - Multidomain Vertically Aligned) verwendet, die in der Literatur ausführlich beschrieben werden, siehe z. B. T. Tsukuda, TFT/LCD: Liquid Crystal Displays Addressed by Thin-Film Transistors, Gordon and Breach 1996, ISBN 2-919875-01-9 und darin zitierte Literatur; SID Symposium 1997, ISSN-0097-966X, Seiten 7 bis 10, 15 bis 18, 47 bis 51, 213 bis 216, 383 bis 386, 397 bis 404 und darin zitierte Literatur.

Clark und Lagerwall (US 4,367,924) konnten zeigen, daß der Einsatz ferroelektrischer Flüssigkristalle (FLC) in sehr dünnen Zellen zu optoelektrischen Schalt- oder Anzeigeelementen führt, die im Vergleich zu den herkömmlichen TN (Twisted Nematic)-Zellen um bis zu einen Faktor 1000 schnellere Schaltzeiten haben (siehe z. B. EP-A-0 032 362). Aufgrund dieser und anderer günstiger Eigenschaften, z. B. der bistabilen Schaltmöglichkeit und des nahezu blickwinkelunabhängigen Kontrasts, sind FLCs grundsätzlich für Anwendungsgebiete wie Computerdisplays und Fernsehgeräte geeignet, wie ein seit Mai 1995 in Japan von Canon vermarkteter Desktop-Monitor zeigt.

Die einzelnen Bildelemente (Pixel) eines LC-Displays sind üblicherweise in einer x,y-Matrix angeordnet, die durch die Anordnung je einer Serie von Elektroden (Leiterbahnen) entlang der Reihen und der Spalten an der Unter- bzw. Oberseite des Displays gebildet wird. Die Kreuzungspunkte der horizontalen (Reihen-) und vertikalen (Spalten-) Elektroden bilden adressierbare Pixel.

Diese Anordnung der Bildpunkte bezeichnet man üblicherweise als eine passive Matrix. Zur Adressierung wurden verschiedene Multiplex-Schemata entwickelt, wie beispielsweise in Displays 1993, Vol. 14, Nr. 2, S. 86-93 und Kontakte 1993 (2), S. 3-14 beschrieben. Die passive Matrixadressierung hat den Vorteil einer einfacheren Herstellung des Displays und damit verbundenen geringen Herstellkosten, jedoch den Nachteil, daß die passive Adressierung immer nur zeilenweise erfolgen kann, was dazu führt, daß die Adressierungszeit des gesamten Bildschirms bei N Zeilen das N-fache der Zeilenadressierungszeit beträgt.

Bei der sogenannten Aktivmatrix-Technologie (AM-LCD) wird üblicherweise ein nichtstrukturiertes Substrat mit einem Aktivmatrix-Substrat kombiniert. An jedem Pixel des Aktivmatrixsubstrates ist ein elektrisch nichtlineares Element, beispielsweise ein Dünnschichttransistor, integriert. Bei dem nichtlinearen Element kann es sich auch um Dioden, Metall-Insulator-Metall u.ä. Elemente handeln, die vorteilhaft mit Dünnschichtverfahren hergestellt werden und in der einschlägigen Literatur beschrieben sind, siehe z. B. T. Tsukuda, TFT/LCD: Liquid Crystal Displays Addressed by Thin-Film Transistors, Gordon and Breach 1996, ISBN 2-919875-01-9 und darin zitierte Literatur.

Aktivmatrix-LCDs werden üblicherweise mit nematischen Flüssigkristallen im TN-(Twisted Nematics), ECB-(Electrically Controlled Birefringence), VA- (Vertically Aligned) oder IPS- (In Plane Switching) Modus betrieben. In jedem Fall wird durch die aktive Matrix an jedem Bildpunkt ein elektrisches Feld individueller Stärke erzeugt, das eine Orientierungsänderung und damit eine Änderung der Doppelbrechung erzeugt, die wiederum im polarisierten Licht sichtbar ist. Ein Nachteil dieser Verfahren ist derzeit noch die mangelnde Videofähigkeit bedingt durch die langen Schaltzeiten nematischer Flüssigkristalle.

Unter anderem aus diesem Grunde wurden auch Flüssigkristallanzeigen, die auf einer Kombination aus ferroelektrischen Flüssigkristallmaterialien und aktiven Matrix-Elementen beruhen, vorgeschlagen, siehe z. B. WO 97/12355, Ferroelectrics 1996, 179, 141-152, oder WO 99/60441.

Für die Verwendung von FLCs in elektrooptischen oder vollständig optischen Bauelementen benötigt man entweder Verbindungen, die geneigte bzw. orthogonale smektische Phasen ausbilden und selbst optisch aktiv sind, oder man kann durch Dotierung von Verbindungen, die zwar solche smektischen Phasen ausbilden, selbst aber nicht optisch aktiv sind, mit optisch aktiven Verbindungen ferroelektrische smektische Phasen induzieren. Die gewünschte Phase soll dabei über einen möglichst großen Temperaturbereich stabil sein.

Zur Erzielung eines guten Kontrastverhältnisses ist eine einheitliche planare Orientierung der Flüssigkristalle nötig. Eine gute Orientierung in der $S_A$ und $S^*_C$-Phase läßt sich z. B. erreichen, wenn die Phasenfolge der Flüssigkristallmischung mit abnehmender Temperatur lautet:

$$\text{Isotrop} \rightarrow N^* \rightarrow S_A \rightarrow S^*_C$$

Voraussetzung ist, daß der Pitch (Ganghöhe der Helix) in der N*-Phase sehr groß (größer 10 μm) oder, noch besser, völlig kompensiert ist (siehe z. B. T. Matsumoto et al., Proc. of the 6th Int. Display Research Conf., Japan Display, Sept. 30 - Okt. 2, 1986, Tokyo, Japan, S. 468-470; M. Murakami et al., ibid. S. 344-347). Dies erreicht man z. B., indem man zu der chiralen Flüssigkristallmischung, die in der N*-Phase z. B. eine linksdrehende Helix aufweist, einen oder mehrere optisch aktive Dotierstoffe, die eine rechtsdrehende Helix induzieren, in solchen Mengen hinzugibt, daß die Helix kompensiert wird.

Für die Verwendung des SSFLCD-Effektes (Surface Stabilized Ferroelectric Liquid Crystal Display) von Clark und Lagerwall zur einheitlichen, planaren Orientierung ist ferner Voraussetzung, daß der Pitch in der smektischen C*-Phase wesentlich größer ist als die Dicke des Anzeigeelementes (Mol. Cryst. Liq. Cryst. 1983, 94, 213 und ibid. 1984, 114, 151).

Die optische Schaltzeit $\tau$ [$\mu$s] ferroelektrischer Flüssigkristallsysteme, die möglichst kurz sein soll, hängt von der Rotationsviskosität des Systems y [mPas], der spontanen Polarisation Ps [nC/cm$^2$] und der elektrischen Feldstärke E [V/m] ab nach der Beziehung

$$\tau \sim \frac{\gamma}{Ps \cdot E}$$

Da die Feldstärke E durch den Elektrodenabstand im elektrooptischen Bauteil und durch die angelegte Spannung festgelegt ist, muß das ferroelektrische Anzeigemedium niedrigviskos sein und/oder eine hohe spontane Polarisation aufweisen, damit eine kurze Schaltzeit erreicht wird.

Schließlich wird neben thermischer, chemischer und photochemischer Stabilität eine kleine optische Anisotropie $\Delta n$ und eine geringe positive oder vorzugsweise negative dielektrische Anisotropie $\Delta\varepsilon$ verlangt (siehe z. B. S. T. Lagerwall et al., "Ferroelectric Liquid Crystals for Displays" SID Symposium, Oct. Meeting 1985, San Diego, Ca, USA).

Die Gesamtheit dieser Forderungen ist nur mit Mischungen aus mehreren Komponenten zu erfüllen. Als Basis (oder Matrix) dienen dabei bevorzugt Verbindungen, die möglichst selbst bereits die gewünschte Phasenfolge I $\rightarrow$ N $\rightarrow$ $S_A$ $\rightarrow$ Sc aufweisen. Weitere Komponenten der Mischung werden oftmals zur Schmelzpunktserniedrigung und zur Verbreiterung der $S_C$- und meist auch N-Phase, zum Induzieren der optischen Aktivität, zur Pitch-Kompensation und zur Anpassung der optischen und dielektrischen Anisotropie zugesetzt, wobei aber beispielsweise die Rotationsviskosität möglichst nicht vergrößert werden soll. Zudem ist es notwendig, daß die unterschiedlichen Komponenten in einem breiten Temperaturbereich untereinander gut mischbar sind.

Da aber die Entwicklung von Flüssigkristallmischungen noch in keiner Weise als abgeschlossen betrachtet werden kann, sind die Hersteller von Displays an den unterschiedlichsten Komponenten für Mischungen interessiert.

Insbesondere werden Flüssigkristallmischungen benötigt, die einerseits einen sehr weiten Arbeits-Temperaturbereich aufweisen, andererseits eine möglichst geringe Schwellspannung besitzen, beispielsweise für den Einsatz in Automobilen, in denen ohne weiteres ein Temperaturbereich von -40°C bis 100°C auftreten kann, aber auch für tragbare Geräte wie Mobiltelefone und Notebook-PCs.

Darüber hinaus scheint der Trend hin zu bewegten Bildern mit nematischen Flüssigkristallen nur dann realisierbar zu sein, wenn man den Elektrodenabstand solcher LCDs verringert, denn die Schaltzeiten sind umgekehrt proportional zum Quadrat des Elektrodenabstandes. Dies wiederum zieht unweigerlich eine Verringerung von Helligkeit und Kontrast nach sich, es sei denn, man verwendet Flüssigkristallmischungen mit einem hohen Wert der optischen Anisotropie ($\Delta n$). Gewöhnlich besitzen Materialien mit hoher optischer Anisotropie jedoch auch hohe Viskositäten und wirken daher dem gesetzten Ziel entgegen. Es besteht daher Nachfrage nach neuen, geeigneten Flüssigkristallmischungen und -mischungskomponenten, die einen hohen Klärpunkt und eine hohe optische Anisotropie besitzen, bei gleichzeitig verhältnismäßig geringer Rotationsviskosität.

Aufgabe der vorliegenden Erfindung ist es daher, neue Komponenten für die Verwendung in smektischen oder nematischen Flüssigkristallmischungen bereit zu stellen, die über hohe positive Werte der dielektrischen Anisotropie kombiniert mit einem günstigen Verhältnis von Viskosität und Klärpunkt verfügen. Darüber hinaus sollen die Verbindungen in hohem Maße licht- und UV-stabil sowie thermisch stabil sein. Ferner sollen sie geeignet sein, eine hohe "voltage holding ratio (VHR)" zu realisieren. Außerdem sollten sie synthetisch gut zugänglich und daher potentiell kostengünstig sein.

Für die Verwendung in flüssigkristallinen Mischungen sind Derivate des Fluorens und Fluorenons bereits aus J. Chem. Soc. 1955, 4359, ibid. 1957, 3228, Pramana Suppl. 1975, 1, 397, Bull. Soc. Chim. Fr. 1975, 2066, Mol. Cryst. Liq. Cryst 1985, 127, 341, ibid. 1985, 129, 17, ibid. 1987, 150B, 361, ibid. 1988, 164, 167, ibid. 1990, 182, 339, DE-A 197 20 289, EP-A 0 113 293, EP-B 0 325 035, JP 05 125055, JP 2000 178211 und EP-A 0 455 219 bekannt.

In der JP 10 236992 läßt sich aus der dort angegebenen allgemeinen Formel unter einer Vielzahl anderer Möglichkeiten auch ein 2,7-disubstituiertes 3,4,5,6-Tetrafluorfluorenderivat oder ein 3,7-disubstituiertes 1,2,8,9-Tetrafluordibenzofuranderivat oder ein 3,7-disubstituiertes 1,2,8,9-Tetrafluordibenzothiophenderivat konstruieren, in der Anmeldung wird jedoch auf die Synthese oder physikalische Eigenschaften entsprechender Verbindungen nicht eingegangen.

1,8-Difluorfluoren-9-on ist beispielsweise aus Nippon Kagaku Kaishi 1989, 12, 2052-2058 (CAN 1990, 112:234935s), 1-Fluorfluoren-9-on beispielsweise aus Chem. Ind. 1961, 179-180 bekannt. Eine Eignung dieser Moleküle oder deren Derivate als Teil einer Komponente von Flüssigkristallmischungen läßt sich daraus jedoch nicht ableiten.

Es wurde nun gefunden, daß Verbindungen der Formel (I)

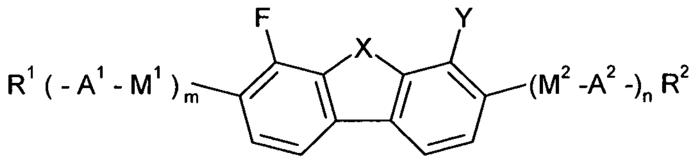

(I)

mit den Bedeutungen:

X ist C=O, O oder S,

Y ist H oder F,

$R^1$, $R^2$ sind gleich oder verschieden
Wasserstoff oder ein geradkettiger oder verzweigter Alkylrest mit 1 bis 16 C-Atomen oder ein geradkettiger oder verzweigter Alkenylrest mit 2 bis 16 C-Atomen, worin

a) eine oder mehrere nicht benachbarte und nicht terminale $CH_2$-Gruppen durch -O-, -C(=O)O-, -O-C(=O)-, -O-C(=O)-O-, -C(=O)- oder -Si$(CH_3)_2$- ersetzt sein können, und/oder
b) eine $CH_2$-Gruppe durch -C≡C-, Cyclopropan-1,2-diyl oder Cyclobutan-1,3-diyl ersetzt sein kann, und/oder
c) ein oder mehrere H-Atome durch F oder Cl ersetzt sein können und/oder
d) die terminale $CH_3$-Gruppe durch eine der folgenden chiralen Gruppen (optisch aktiv oder racemisch) ersetzt sein kann:

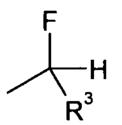 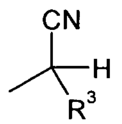 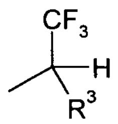 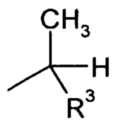

wobei $R^3$ ein geradkettiger oder verzweigter Alkylrest mit 1 bis 12 C-Atomen oder ein geradkettiger oder verzweigter Alkenylrest mit 2 bis 12 C-Atomen ist, worin eine oder mehrere nicht benachbarte und nicht terminale $CH_2$-Gruppen durch -O-ersetzt sein können und/oder ein oder mehrere H-Atome durch F oder Cl ersetzt sein können,

mit der Maßgabe, daß nur einer von $R^1$, $R^2$ gleich Wasserstoff sein kann,

$M^1$, $M^2$ sind gleich oder verschieden -C(=O)-O-, -O-C(=O)-, -$CH_2$-O-, -O-$CH_2$-, -$CF_2$-O-, -O-$CF_2$-, -CH=CH-, -CF=CF-, -C≡C-, -$CH_2$-$CH_2$-C(=O)-O-, -O-C(=O)-$CH_2$-$CH_2$-, -$CH_2$-$CH_2$-, -$CF_2$-$CF_2$-, -$(CH_2)_4$- oder eine Einfachbindung,

$A^1$, $A^2$ sind gleich oder verschieden 1,4-Phenylen, worin ein oder zwei H-Atome durch F, Cl oder CN ersetzt sein können, Pyridin-2,5-diyl, worin ein H-Atom durch F ersetzt sein kann, Pyrimidin-2,5-diyl, 1,4-Cyclohexylen, worin ein oder zwei H-Atome durch $CH_3$ oder F ersetzt sein können, 1-Cyclohexen-1,4-diyl, worin ein H-Atom durch $CH_3$ oder F ersetzt sein kann, oder 1,3-Dioxan-2,5-diyl,

m, n sind unabhängig voneinander 0 oder 1, in Summe jedoch nicht größer als 1,

diesen Anforderungen in besonderer Weise genügen.
Liegen an mehreren der genannten Positionen Ersetzungen vor, so sind sie voneinander unabhängig aus den genannten Ersetzungen ausgewählt und können daher gleich oder verschieden sein. Es können damit in der Verbindung auch mehrere unterschiedliche der genannten Ersetzungen an unterschiedlichen Positionen gleichzeitig vorliegen.
Bevorzugt sind die Verbindungen der Formel (I) mit den Bedeutungen:

X ist C=O oder O,

$R^1$, $R^2$ sind gleich oder verschieden
ein geradkettiger oder verzweigter Alkylrest mit 1 bis 14 C-Atomen oder ein geradkettiger oder verzweigter Alkenylrest mit 2 bis 14 C-Atomen, worin eine oder zwei nicht benachbarte und nicht terminale $CH_2$-Gruppen durch -O- ersetzt sein können und/oder ein oder mehrere H-Atome durch F ersetzt sein können,

$R^3$ ist ein geradkettiger oder verzweigter Alkylrest mit 1 bis 10 C-Atomen, worin eine nicht terminale $CH_2$-Gruppe durch -O- ersetzt sein kann,

$M^1$, $M^2$ sind gleich oder verschieden
$-CH_2-O-$, $-O-CH_2-$, $-CH_2-CH_2-$ oder eine Einfachbindung,

$A^1$, $A^2$ sind gleich oder verschieden
1,4-Phenylen, worin ein oder zwei H-Atome durch F ersetzt sein können oder 1,4-Cyclohexylen,

m, n sind unabhängig voneinander 0 oder 1, in Summe jedoch nicht größer als 1.

Besonders bevorzugt sind die folgenden Verbindungen der Formeln (I i) bis (I q)

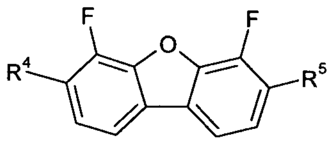

(I i)

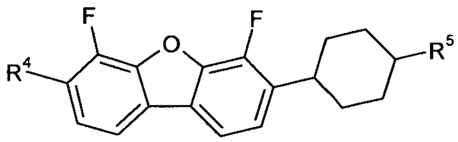

(I k)

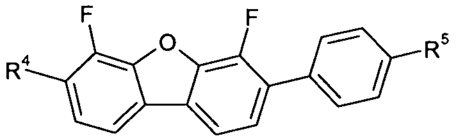

(I l)

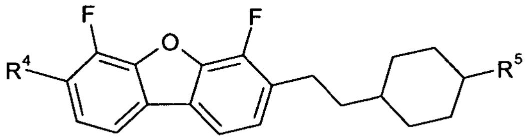

(I m)

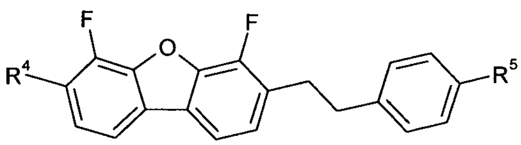

(I n)

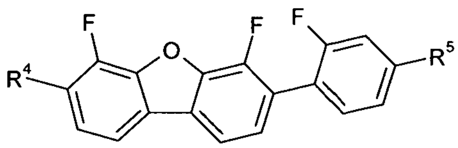

(I o)

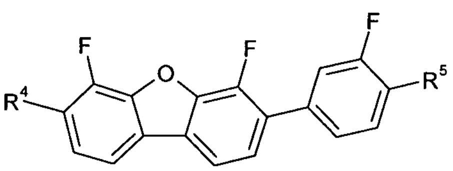

(I p)

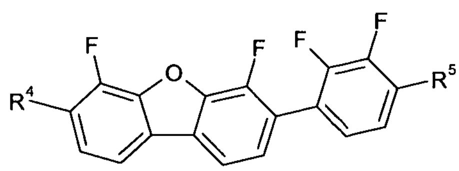

(I q)

mit den Bedeutungen:

$R^4$ und $R^5$ sind gleich oder verschieden ein geradkettiger Alkyl-, Alkenyl- oder Alkoxyrest mit 2 bis 12 C-Atomen.

Ganz besonders bevorzugt sind Verbindungen der Formeln (I i), (I k) und (I 1) mit den Bedeutungen:

$R^4$, $R^5$ sind gleich oder verschieden ein geradkettiger Alkyl-, Alkenyl- oder Alkoxyrest mit 2 bis 10 C-Atomen.

Mit der Bereitstellung von Verbindungen der Formel (I) wird ganz allgemein die Palette der flüssigkristallinen Substanzen, die sich unter verschiedenen anwendungstechnischen Gesichtspunkten zur Herstellung flüssigkristalliner Gemische eignen, erheblich erweitert.

In diesem Zusammenhang besitzen die Verbindungen der Formel (I) einen breiten Anwendungsbereich. In Abhängigkeit von der Auswahl der Substituenten können sie anderen Verbindungsklassen zugesetzt werden, um beispielsweise die dielektrische und/oder optische Anisotropie eines solchen Dielektrikums zu beeinflussen. Auch können sie dazu dienen, dessen Schwellenspannung und/oder dessen Viskosität zu optimieren.

Besonders geeignet sind die Verbindungen der Formel (I), um schon in geringen Zumischmengen die dielektrische Anisotropie ($\Delta\varepsilon$) in Richtung auf höhere negative Werte zu beeinflussen.

Gegenstand der Erfindung ist somit neben Verbindungen der Formel (I) auch die Verwendung dieser Verbindungen als Komponenten flüssigkristalliner Mischungen.

Die Verbindungen der Formel (I) können in Flüssigkristallmischungen eingesetzt werden. Sie eignen sich im Falle von nematischen Mischungen besonders für "Active Matrix Displays" (AM-LCD) (siehe z. B. C. Prince, Seminar Lecture Notes, Volume I, p. M-3/3-M-22, SID International Symposium 1997, B. B: Bahadur, Liquid Crystal Applications and Uses, Vol. 1, p. 410, World Scientific Publishing, 1990, E. Lüder, Recent Progress of AMLCD's, Proceedings of the 15th International Display Research Conference , 1995, p. 9-12) und "in-plane-switching Displays" (IPS-LCD), im Falle von smektischen Flüssigkristallmischungen für geneigt smektische (ferroelektrische oder antiferroelektrische) Displays. Im Fall von ferroelektrischen Mischungen eignen sich die erfindungsgemäßen Verbindungen der Formel (I) insbesondere zur Verwendung in Displays, die im Inverse-Mode (siehe z. B.: J. C. Jones, M. J. Towler, J. R. Hughes, Displays 1993, 14 , Nr. 2, 86-93; M. Koden, Ferroelectrics 1996, 179, 121-129) betrieben werden.

Weitere Komponenten von Flüssigkristallmischungen, die erfindungsgemäße Verbindungen der Formel (I) enthalten, werden vorzugsweise ausgewählt aus den bekannten Verbindungen mit smektischen und/oder nematischen und/oder cholesterischen Phasen. In diesem Sinne geeignete Mischungskomponenten sind insbesondere in WO 00/36054, WO 97/04039, DE-A-195 31 165 sowie EP-A-0 893 424 aufgeführt, auf die hiermit ausdrücklich Bezug genommen wird.

Gegenstand der Erfindung sind auch Flüssigkristallmischungen, die mindestens eine Verbindung der Formel (I), vorzugsweise in einer Menge von 1 bis 40 Gew.-%, bezogen auf die Flüssigkristallmischung, enthalten. Vorzugsweise enthalten die Mischungen mindestens 3 weitere Komponenten bzw. (flüssiglcristalline) Verbindungen. Gegenstand der Erfindung sind darüber hinaus auch elelctrooptische Anzeigeelemente (Flüssigkristallanzeigen, Displays), die die erfindungsgemäßen Mischungen enthalten.

Bevorzugt sind Flüssigkristallanzeigen, die die erfindungsgemäßen nematischen oder smektischen (ferroelektrischen oder antiferroelelctrischen) Mischungen in Kombination mit Aktiv-Matrix-Elementen enthalten.

Ebenfalls bevorzugt sind Flüssigkristallanzeigen, die die erfindungsgemäßen ferroelektrischen Flüssigkristallmischungen enthalten und im sogenannten Inverse- oder $\tau V_{(min)}$-Mode betrieben werden.

Die erfindungsgemäßen Anzeigeelemente (Displays) sind üblicherweise so aufgebaut, daß eine Flüssigkristallschicht beiderseitig von Schichten eingeschlossen ist, die üblicherweise, in dieser Reihenfolge ausgehend von der LC-Schicht, mindestens eine Orientierungsschicht, Elektroden und eine Begrenzungsscheibe (z. B. aus Glas) sind. Darüber hinaus können sie Abstandshalter, Kleberahmen, Polarisatoren sowie für Farbdisplays dünne Farbfilterschichten enthalten. Weitere mögliche Komponenten sind Antireflex-, Passivierungs-, Ausgleichs- und Sperrschichten sowie elektrisch-nichtlineare Elemente, wie Dünnschichttransistoren (TFT) und Metall-Isolator-Metall-(MIM)-Elemente. Im Detail ist der Aufbau von Flüssigkristalldisplays bereits in einschlägigen Monographien beschrieben (siehe z. B. E. Kaneko, "Liquid Crystal TV Displays: Principles and Applications of Liquid Crystal Displays", KTK Scientific Publishers, 1987).

Die Schaltcharakteristika einer FLC-Vorrichtung können im allgemeinen durch ein Diagramm dargestellt werden, bei dem die Treiberspannung (V) horizontal und die Breite der Ansteuerpulse ($\tau$, Zeit) vertikal aufgetragen sind (siehe z. B. J. C. Jones, M. J. Towler, J. R. Hughes, Displays 1993, 14 , Nr. 2, 86-93, Abb. 4, 8, 10 und 11). Eine Schaltkmve wird experimentell bestimmt und teilt die V, $\tau$-Fläche in einen schaltenden und einen nicht schaltenden Bereich. Üblicherweise verkürzt sich bei Erhöhung der Spannung die Pulsbreite. Dieses Verhalten kennzeichnet den sogenannten Normal-Mode (siehe z. B. Jones, Abb. 4). Bei geeigneten Materialien weist die $V\tau$-Kurve jedoch ein Minimum auf (bei der Spannung $V_{(min)}$), wie z. B. bei Jones in den Abbildungen 8, 10 und 11 zu sehen. Dieses Minimum kommt durch Überlagerung von dielektrischer und ferroelektrischer Verdrillung zustande. FLC-Vorrichtungen werden im Inverse-Mode betrieben, wenn die Summe der Zeilen- und Spalten-Treiberspannung im Arbeitstemperaturbereich höher als das Minimum auf der $V\tau$-Kurve sind, d. h. $V_{(Zeile)} + V_{(Spalte)} > V_{(min)}$.

Beispielhaft sind in den nachfolgenden Schemata 1 bis 7 mögliche Synthesewege zu Verbindungen der Formel (I) angegeben, wobei auch andere Verfahren denkbar und möglich sind.

Folgende Abkürzungen werden verwendet:

| | |
|---|---|
| *n*-BuLi | *n*-Buthyllithium |
| DCC | Dicyclohexylcarbodiimid |
| DEAD | Diethylazodicarboxylat (Azodicarbonsäurediethylester) |
| DMAP | 4-(Dimethylamino)pyridin |
| DME | Dimethoxyethan |
| DMF | N,N-Dimethylformamid |
| KO*t*Bu | Kalium-*tert*-butylat |
| LiTMP | Lithium-2,2,6,6-tetramethylpiperidid |
| MTBE | *tert*-Butylmethylether |
| 4-TsOH | 4-Toluolsulfonsäure |

Schema 1

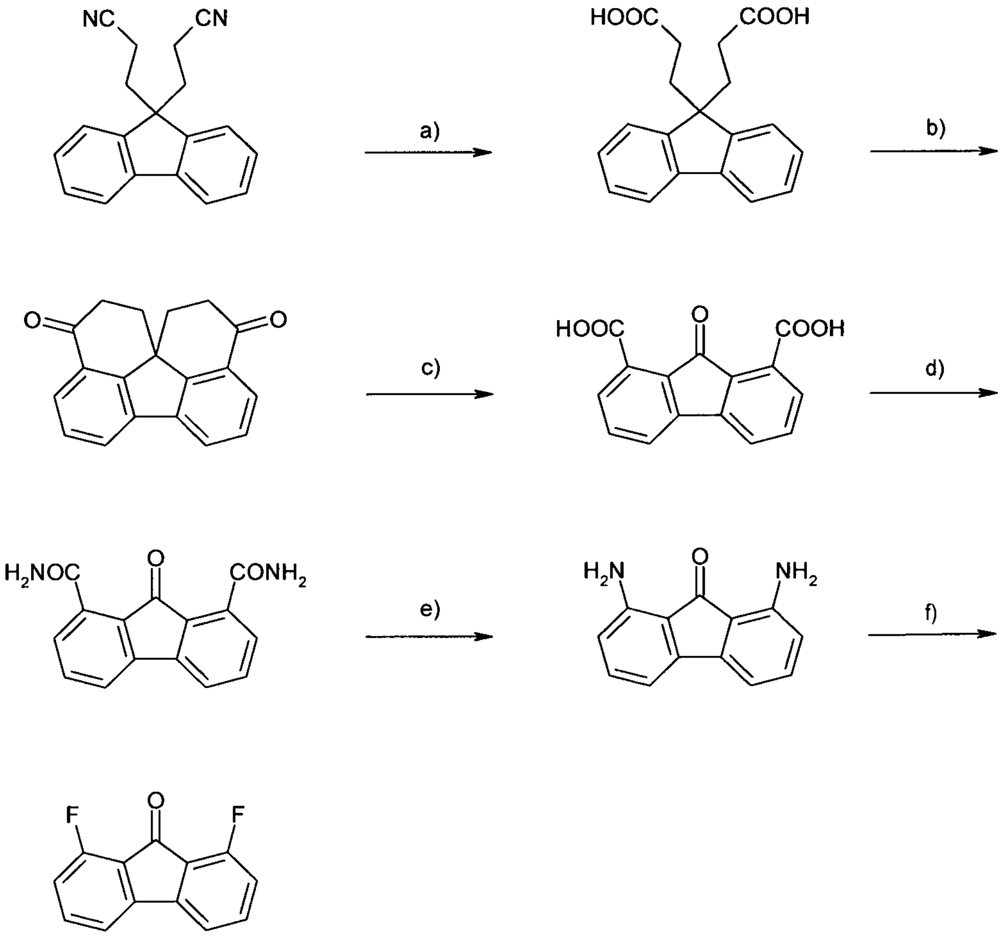


a) $H_2SO_4/H_2O$ b) Polyphosphorsäure c)$KMnO_4$
d) 1. $SOCl_2$ 2. $NH_3$ e) $NaOBr/H_2O$
f) 1. $NaNO_2/HCl/H_2O$ 2. $HBF_4/H_2O$ 3. $\Delta$ g) $Et_3SiH/CF_3COOH$

Die Synthese von 1,8-Difluorfluoren-9-on [127375-32-0] erfolgt wie in Nippon Kagaku Kaishi 1989, 12, 2052 und Yamaguchi Daigaku Kogakubu Kenkyu Hokoku 1995, 45, 281 beschrieben (Schema 1). Käufliches 9,9-Bis(2-Cyanoethyl)fluoren (9,9-Fluorendipropionitril [4425-97-2]; hergestellt durch Michael-Addition von Acrylnitril an Fluoren) wird verseift, und die erhaltene Fluoren-9,9-dipropansäure [4425-95-0] (siehe auch: J. Org. Chem. 1968, 33,2575) wird mit Polyphosphorsäure zum 1,2,11,12-Tetrahydro-3H,10H-benzo[hi]fluoranthen-3,10-dion [98804-54-7] cyclisiert. Oxidation liefert 9-Oxofluoren-1,8-dicarbonsäure [94024-99-4],die über das entsprechende Carbonsäurechlorid zum 9-Oxofluoren-1,8-dicarboxamid [127375-28-4] (allgemeine Arbeitsvorschrift: Organikum, VEB Deutscher Verlag der Wissenschaften, 15. Aufl., Berlin 1984, Kap. 7.1.5.4 und 7.1.5.2) umgesetzt wird. Hofmann-Abbau (allgemeine Arbeitsvorschrift: ibid., Kap. 9.1.2.1) liefert 1,8-Diaminofluoren-9-on [127375-29-5], das in das entsprechende Bis(Diazoniumtetrafluoroborat) überführt und durch thermische Zersetzung nach Balz-Schiemann 1,8-Difluorfluoren-9-on

[127375-32-0] liefert (siehe auch: J. Am. Chem. Soc. 1974, 96, 3536).

Analog läßt sich 1,8-Difluorfluoren-9-on auch ausgehend von Fluoren-1,8-dicarbonsäure [88830-26-6] (hergestellt durch Acylierung von Fluorenon mit Dimethylcarbamoylchlorid nach Ref. Zh., Khim. 1983, Abstr. No. 16Zh185) an Stelle von 9-Oxofluoren-1,8-dicarbonsäure erhalten (Schema 2).

Schema 2

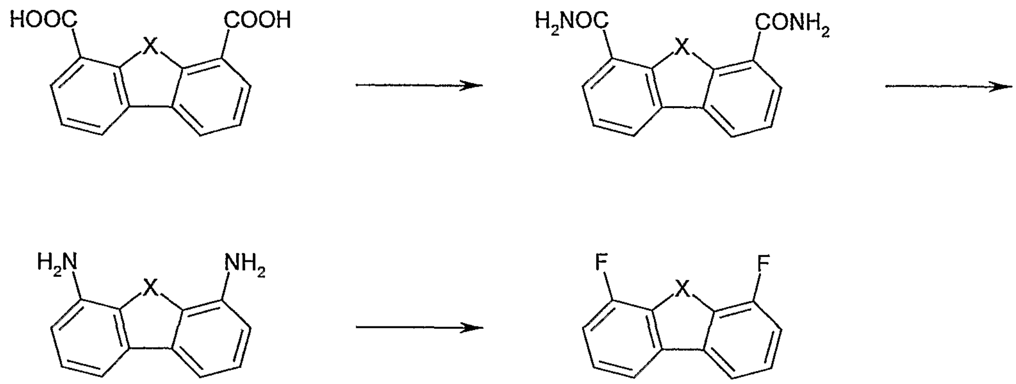

Dibenzofuran-4,6-dicarbonsäure [88818-47-7] und Dibenzofuran-4-carbonsäure [2786-05-2] (erhalten durch Carboxylierung von Dibenzofuran: Tetrahedron: Asymmetry 1999, 10, 2417; J. Am. Chem. Soc. 1998, 120, 3074; Tetrahedron 1997, 53, 17425; J. Chem. Soc., Chem. Commun. 1995, 15, 1515) sowie Dibenzothiophen-4,6-dicarbonsäure [69747-72-4] und Dibenzothiophen-4-carbonsäure [2786-08-5] (erhalten durch Carboxylierung von Dibenzothiophen: Acta Pharm. Suec. 1978, 15, 337) lassen sich analog Schema 1 zu 4,6-Difluordibenzofuran, 4-Fluorbenzofuran, 4,6-Difluorbenzothiophen und 4-Fluordibenzothiophen umsetzen (Schema 2).

Durch Metallierung von 1,8-Difluorfluoren in Position 2 bzw. 7 oder 4,6-Difluordibenzofuran in Position 3 bzw. 7 in einer Mischung aus *n*-Butyllithium und Kalium-*tert*-butylat in THF bei Temperaturen ≤-70°C (analog: Synlett 1990, 747), anschließende Umsetzung mit Elektrophilen (z. B. Trimethylborat, Aldehyden, Ketonen, 4-*n*-Alkylcyclohexanonen, Iod, Brom) und nachfolgende, dem Fachmann bekannte Umsetzungen von beispielsweise erhaltener 1,8-Difluorfluoren-2-boronsäure, 1,8-Difluorfluoren-2-ol oder 2-(1-Hydroxyalkyl)- bzw. 2-(1-Hydroxycyclohexyl)-1,8-difluorfluoren sind verschiedene, in Stellung 2 substituierte 1,8-Difluorfluorene erhältlich (Schema 3).

Schema 3

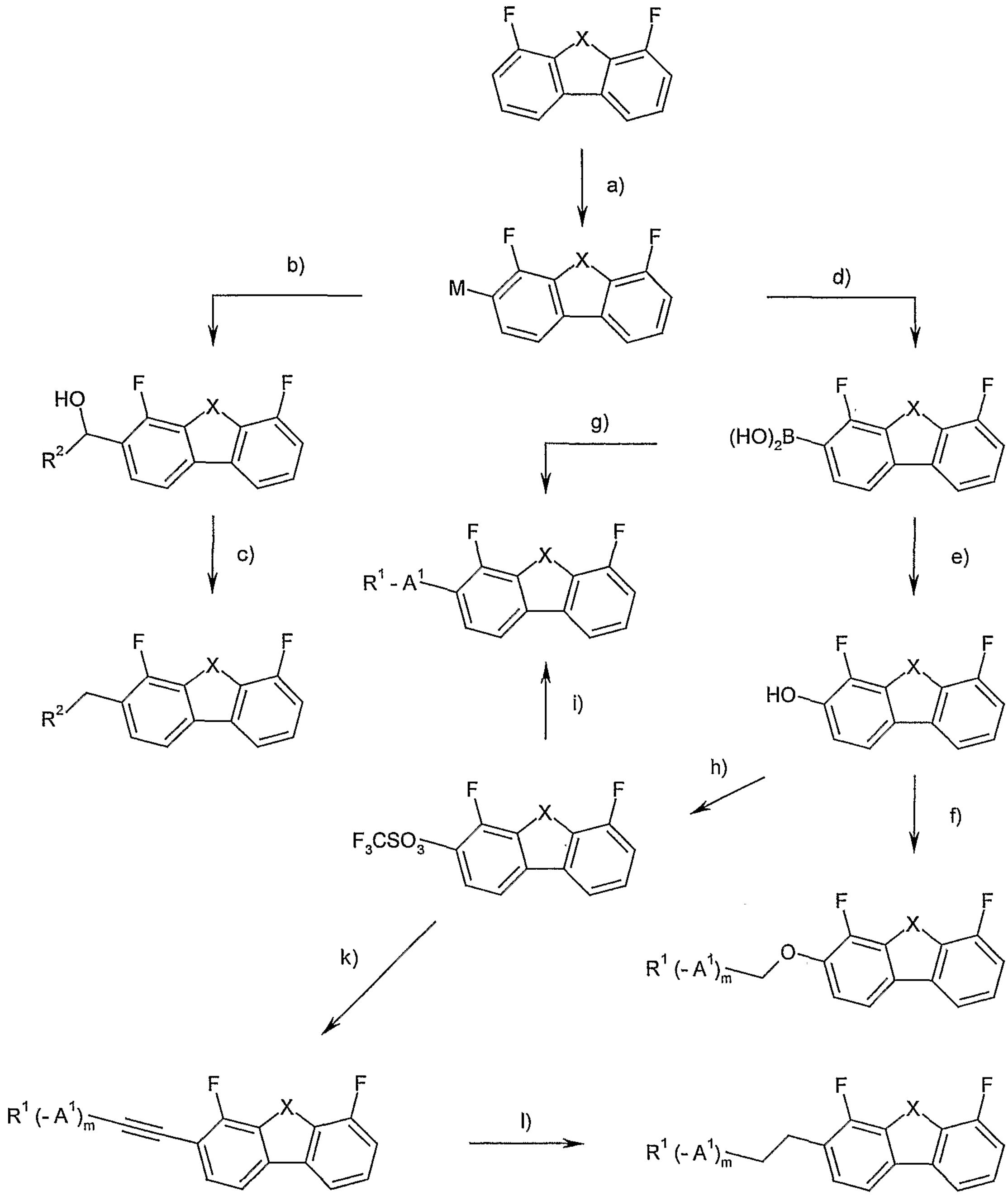


M = Li, K a) *n*-BuLi/KOtBu/THF/Hexan oder *n*-BuLi/KO*t*Bu/THF/Hexan oder *sec*-BuLi/THF/Cyclohexan oder LiTMP/THF/Hexan b) $R^1$-$CH_2CHO$ 2. $H_3O^+$ c) 1. 4-TsOH/Toluol 2. $H_2$/Pd-C/Toluol d) 1. $B(OMe)_3$ 2. $H_3O^+$ e) $H_2O_2$/MTBE f) $R^1(-A^1)_m$ $CH_2OH$/DEAD/$PPh_3$/THF oder $R^1(-A^1)_m$-$CH_2Br$/$K_2CO_3$/MEK g) $R^1(-A^1)_m$-Hal/$Pd(PPh_3)_4$/$Na_2CO_3$/Toluol/EtOH/$H_2O$ h) $(F_3CSO_2)_2O$/Pyridin i) $R^1(-A^1)_m$-$B(OH)_2$/$Pd(PPh_3)_4$/$Na_2CO_3$/Toluol/EtOH/$H_2O$ k) $R^1(-A^1)_m$-C≡CH/$Pd(PPh_3)_2Cl_2$/CuI/$NEt_3$ 1) $H_2$/Pd-C/THF

Die Gruppe Hal bedeutet Cl, Br, I oder $OSO_2CF_3$. In den Schemata 4 bis 7 bedeutet die Gruppe $R^x$ die Gruppierung $R^1(-A^1-M^1)_m$ oder eine geeignete, gegebenenfalls geschützte Vorstufe hiervon (beispielsweise Br oder $OSO_2CF_3$ im Fall Hal = I; Benzyloxy; Tetrahydopyranyloxy; *tert*-Butyldiphenylsilanyloxy), die in späteren Schritten nach an sich bekannten, dem Fachmann geläufigen Methoden in diese Gruppe überführt werden kann.

In Position 3 durch geeignete $R^x$ substituierte 4,6-Difluordibenzofurane können durch Metallierung der Position 7 (analog: Synlett 1990, 747, ibid. 1991, 119, Tetrahedron Lett. 1996, 37, 6551) und nachfolgende Umsetzung mit Elektrophilen (z. B. Trimethylborat, DMF, $CO_2$, *n*-Alkylaldehyden, 4-*n*-Alkylcyclohexanonen, Iod, Brom) zu Vorstufen umgesetzt werden, aus denen sich die erfindungsgemäßen Verbindungen der Formel (I) herstellen lassen (Schemata

4 bis 7).

Schema 4

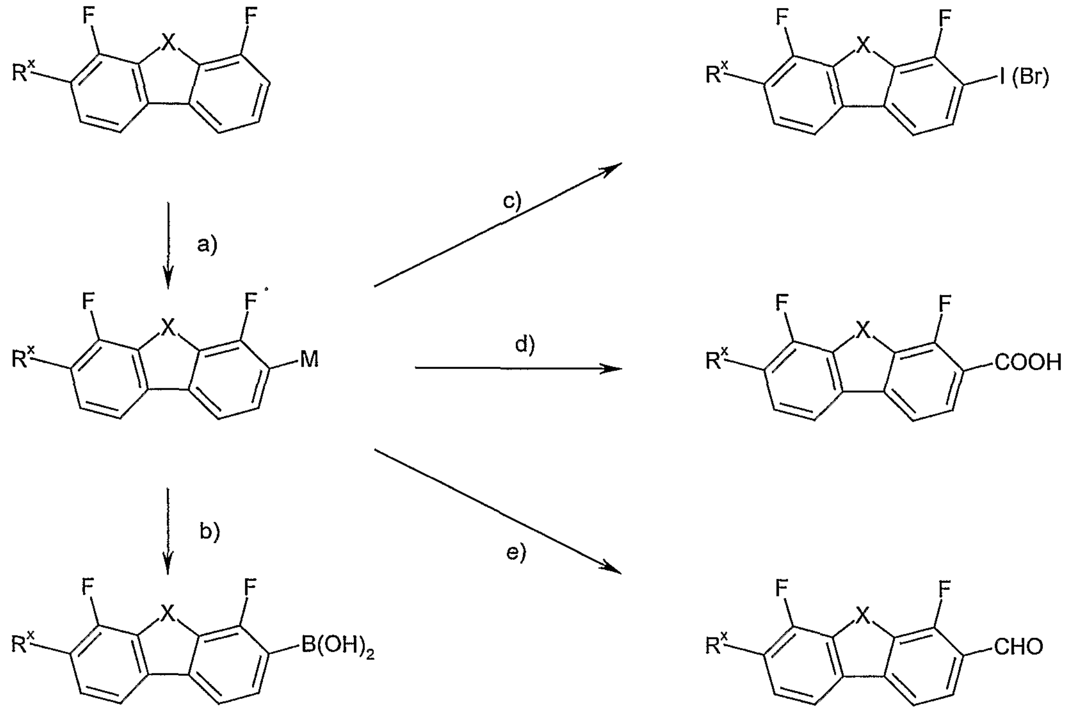


M = Li, K a) *n*-BuLi/THF/Hexan oder *n*-BuLi/KO*t*Bu/THF/Hexan oder *sec*-BuLi/THF/Cyclohexan oder LiTMP/THF/Hexan b) 1. $B(OMe)_3$ 2. $H_3O^+$ c) $I_2$ (bzw. $Br_2$) d) 1. $CO_2$ 2. $H_3O^+$ e) 1. DMF 2. $H_3O^+$

Schema 5

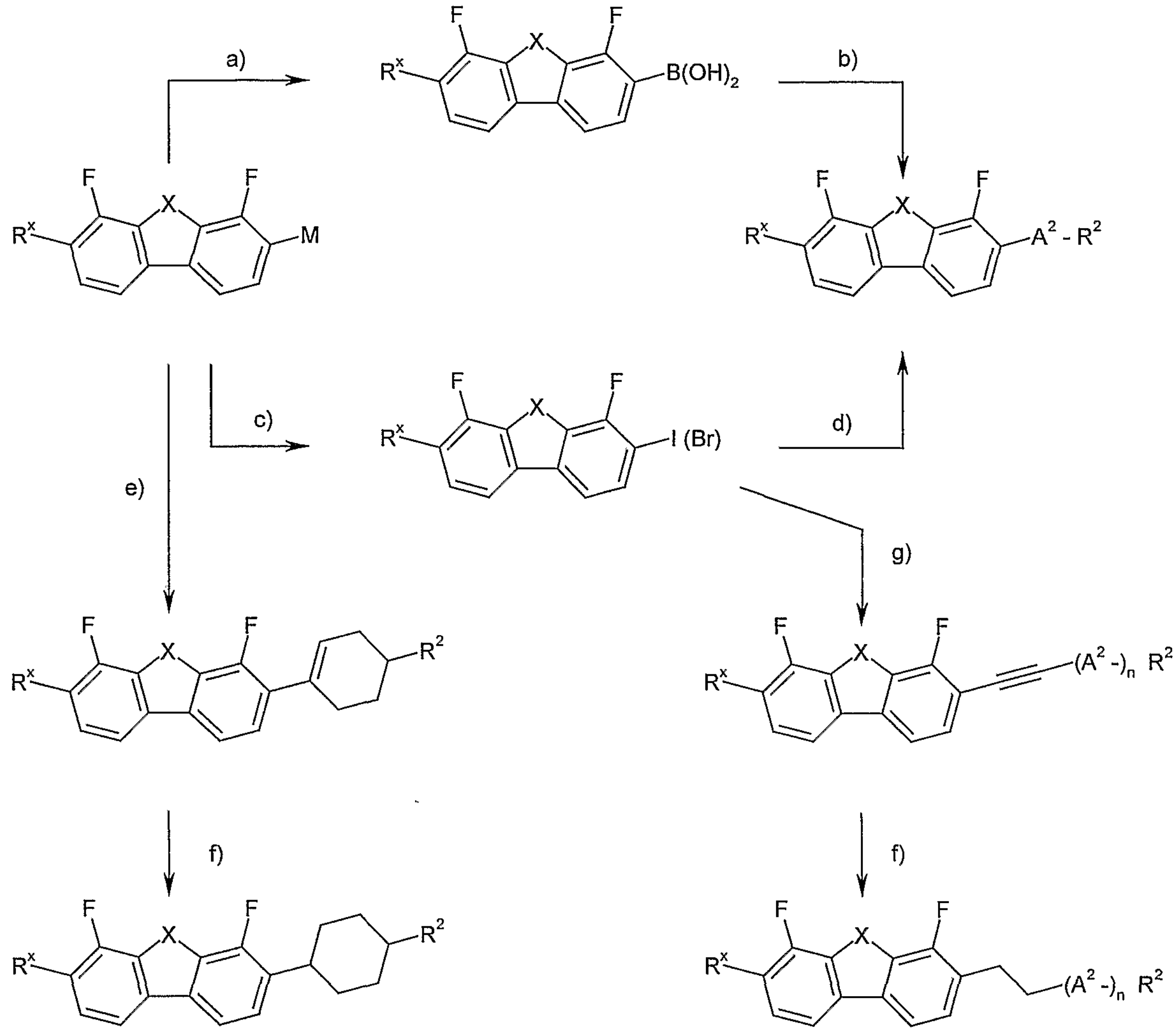


a) 1. $B(OMe)_3$ 2. $H_3O^+$ b) $R^2$-$A^2$-Hal/$Pd(PPh_3)_4$/$Na_2CO_3$/Toluol/EtOH/$H_2O$ c) $I_2$ (oder $Br_2$) d) $R^2$-$A^2$-$B(OH)_2$/$Pd(PPh_3)_4$/$Na_2CO_3$/Toluol/EtOH/$H_2O$ e) 1. 4-($R^2$)-Cyclohexanon 2. $H_3O^+$ 3. 4-TsOH/Toluol f) $H_2$/Pd-C/THF g) $R^2$-$(A^2)_n$-C≡CH/$Pd(PPh_3)_2Cl_2$/CuI/$NEt_3$

Schema 6

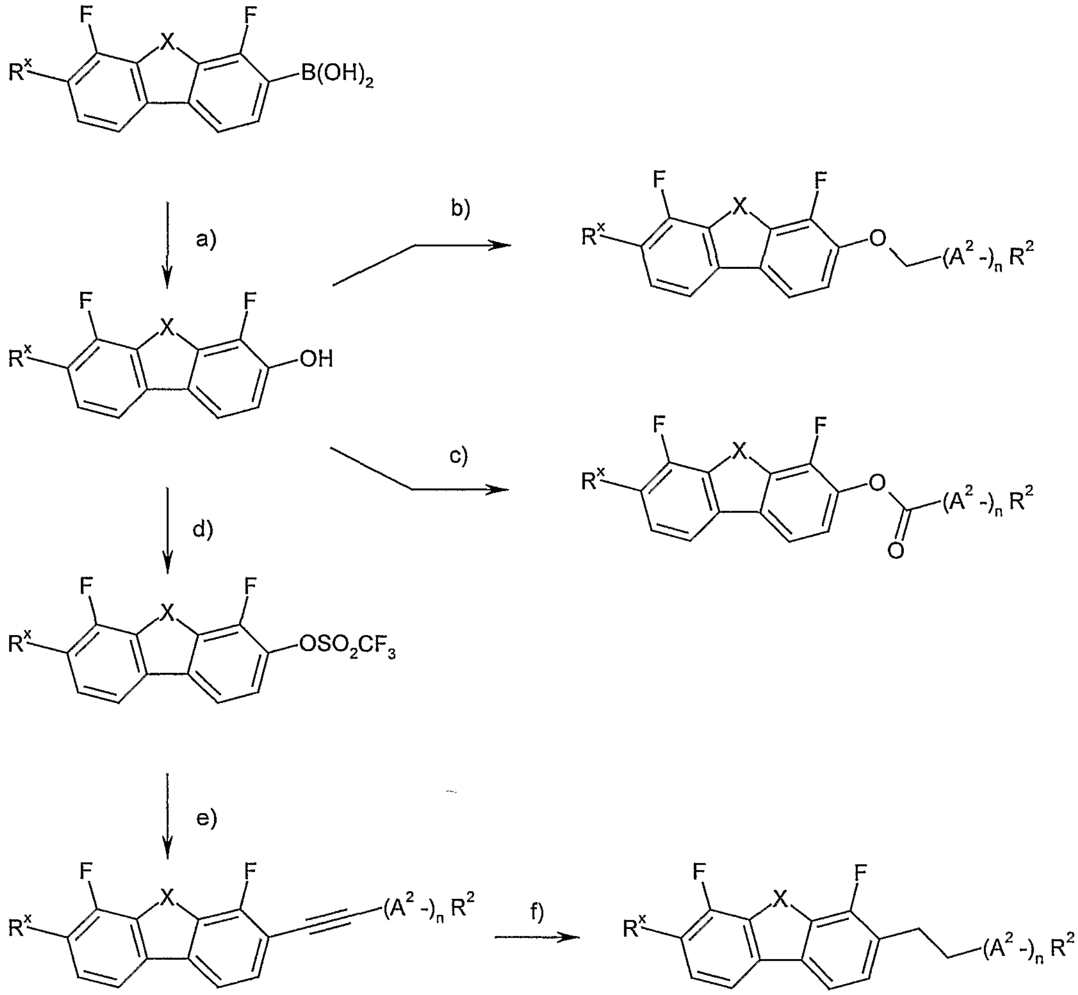


a) $H_2O_2$/MTBE b) $R^2(-A^2)_n-CH_2OH$/DEAD/$PPh_3$/THF oder $R^2(-A^2)_n-CH_2Br/K_2CO_3$/MEK c) $R^2(-A^2)_n$-COOH/DCC/DMAP/$CH_2Cl_2$ d) $(F_3CSO_2)_2O$/Pyridin e) $R^2(-A^2)_n-C\equiv CH/Pd(PPh_3)_2Cl_2$/CuI/$NEt_3$ f) $H_2$/Pd-C/THF

Schema 7

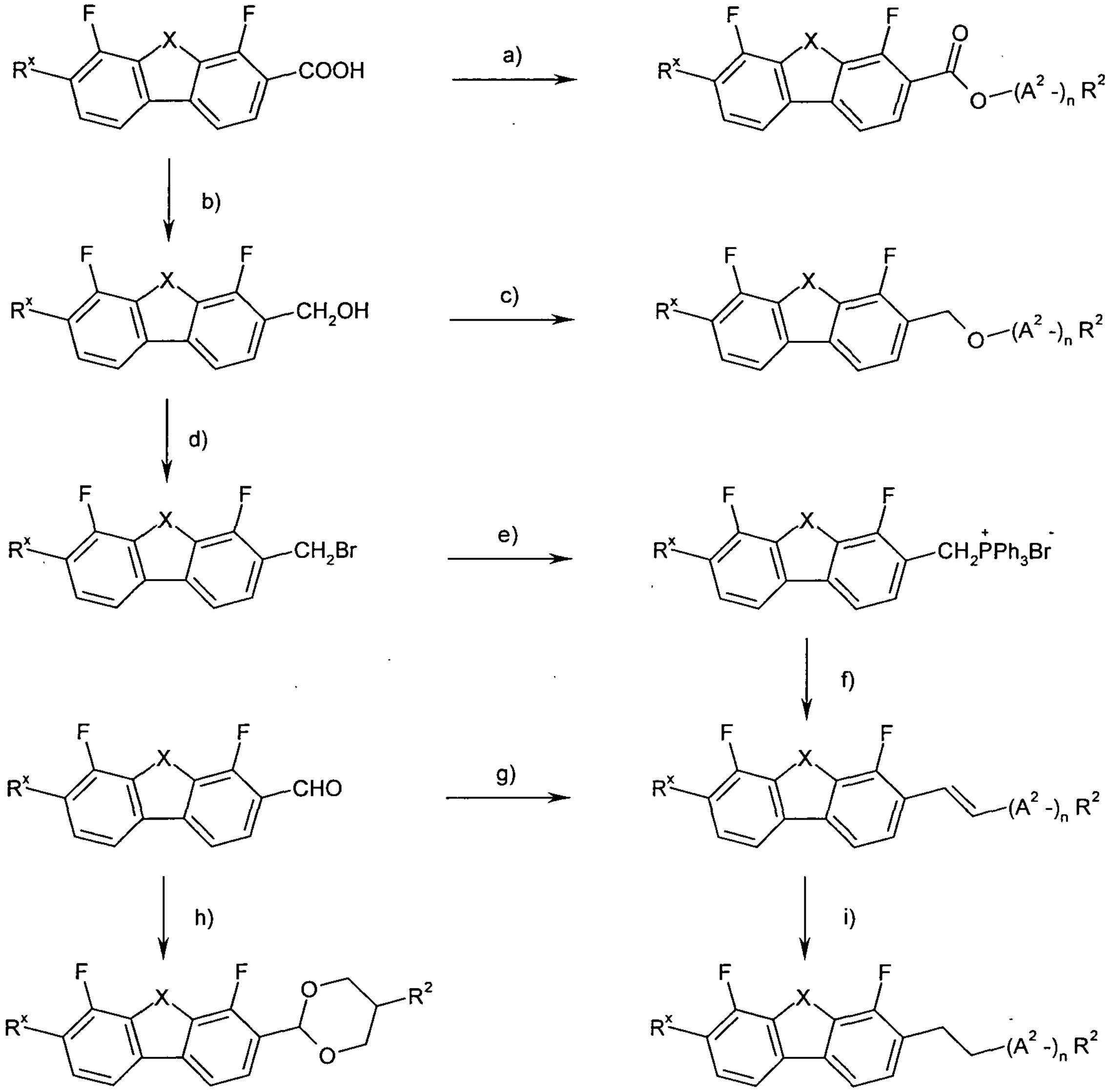


a) $R^2(-A^2)_n$-OH/DCC/DMAP/$CH_2Cl_2$ b) $LiAlH_4$/THF 2. $H_3O^+$ c) $R^2(-A^2)_nOH$/DEAD/$PPh_3$/THF d) $PBr_3$/$CHCl_3$ e) $PPh_3$/Xylol f) $R^2(-A^2)_n$-CHO/KO*t*Bu/THF g) $R^2(-A^2)_nCH_2P(Ph_3)Br$/KO*t*Bu/THF h) $R^2$-$CH(CH_2OH)_2$/4-TsOH/Toluol i) $H_2$/Pd-C/THF

Die Herstellung der für die Synthesen nach Schema 3 bis 7 benötigten, beispielsweise alkyl-, alkenyl- oder alkoxy-substituierten, ggf. zusätzlich fluorierten Benzoesäuren, Cyclohexancarbonsäuren und Phenylacetylene (Schemata 3, 5 und 6), Phenylboronsäuren, Pyridylboronsäuren (Schemata 3 und 5), Brombenzole, Brompyrimidine (Schemata 3 und 7), Cyclohexanone (Schema 5) und 2-Alkylpropan-1,3-diole (Schemata 5 und 7) und deren Umsetzungen sind dem Fachmann bekannt und beispielsweise in WO 96/00710, WO 96/30344, Liq. Cryst. 1995, 18, 1, Mol. Cryst. Liq. Cryst. 1991, 204, 43, Liq. Cryst. 1997, 23, 389, Synthesis 1996, 589, WO 92/11241, EP-A-0 665 825 und J. Mater. Chem. 1999, 9, 1669 beschrieben. Entsprechend substituierte Benzylalkohole und (Hydroxymethyl)-cyclohexane $R^2$-$A^2$-$CH_2OH$ lassen sich beispielsweise aus den entsprechenden Benzoesäuren bzw. Cyclohexancarbonsäuren $R^2$-$A^2$-COOH durch Reduktion mit Lithiumaluminiumhydrid (allg. Arbeitsvorschrift: Organikum, VEB Deutscher Verlag der Wissenschaften, 15. Aufl., Berlin 1984, Kap. 7.3.4) erhalten. Deren Bromierung mit Phosphortribromid (analog J. Org. Chem. 1984, 49, 2534-2540) liefert die in Schema 5 benötigten Benzylbromide bzw. (Brommethyl)-cyclohexane $R^2$-$A^2$-$CH_2Br$. Anschließende Umsetzung mit Triphenylphosphin in Xylol liefert die in Schema 4 erwähnten Triphenylphosphoniumbromide $R^2$-$A^2$-$CH_2$-$P(Ph_3)Br$. Entsprechend substituierte Benzaldehyde und Cyclohexancarboxaldehyde $R^2$-$A^2$-CHO sind beispielsweise durch Reduktion des jeweiligen Carbonsäureesters $R^2$-$A^2$-COOR (Bull. Korean. Chem. Soc. 1999, 20, 1373) oder Oxidation der zuvor genannten Benzylalkohole und (Hydroxymethyl)-cyclohexane $R^2$-$A^2$-$CH_2OH$ (Tetrahedron Lett. 1968, 30, 3363) erhältlich.

Erfindungsgemäße Verbindungen der Formel (I) mit 1-Cyclohexen-1,4-diyl- oder 2-Fluor-1-cyclohexen-1,4-diyl- oder 4-Fluor-3-cyclohexen-1-yl-Einheit werden wie in Liq. Cryst. 1997, 23, 69, DE-A-44 27 266, DE-A-196 07 996, DE-A-195 28 665 und EP-A-0 736 513 beschrieben hergestellt. Was die Synthese spezieller Reste $R^1$ und $R^2$ angeht, sei zusätzlich beispielsweise verwiesen auf EP-A-0 355 008 (für Verbindungen mit siliciumhaltigen Seitenketten), US 4,798,680 (für optisch aktive Verbindungen mit 2-Fluoralkyloxy-Einheit), EP-A-0 552 658 (für Verbindungen mit Cyclohexylpropionsäureresten) und EP-A-0 318 423 (für Verbindungen mit Cyclopropylgruppen in der Seitenkette).

Die Synthese von 9,9-Difluorfluoren ausgehend von Fluorenon ist in J. Org. Chem. 1981, 46, 3917, J. Chem. Soc., Chem. Commun. 1995, 177, Synlett 1991, 191 und Tetrahedron 1996, 52, 9 beschrieben.

Die Erfindung wird durch die nachstehenden Beispiele näher erläutert.

Vergleichsbeispiel 1:

2-Butyloxy-1,8-difluorfluoren

Eine Lösung von 94 mmol *n*-Butyllithium (1,6 M Lösung in Hexan) in 50 ml trockenem Tetrahydrofuran wird auf ≤ -70°C gekühlt und unter Schutzgasatmosphäre tropfenweise mit einer Lösung von 94 mmol Kalium-*tert*-butylat in 100 ml des gleichen Lösemittels versetzt. Nach 15-30 min Rühren tropft man eine Lösung von 78 mmol 1,8-Difluorfluoren in 200 ml Tetrahydrofuran so zu, daß die Innentemperatur stets unterhalb -70°C liegt. Anschließend rührt man noch 2 h bei dieser Temperatur nach. Nun tropft man eine Lösung von 117 mmol Trimethylborat in 100 ml Tetrahydrofuran zu und läßt langsam auftauen. Die Reaktionsmischung wird bei -10°C mit 50 ml Wasser versetzt und mit konzentrierter Salzsäure auf pH 1 angesäuert. Man trennt die Phasen, wäscht die organische Phase mit gesättigter Natriumchloridlösung, trocknet mit Natriumsulfat und entfernt die Lösemittel im Vakuum. Man erhält 1,8-Difluorfluoren-2-boronsäure, die ohne Reinigung weiter umgesetzt wird.

Zu einer Suspension von 54 mmol 1,8-Difluorfluoren-2-boronsäure in 120 ml *tert*-Butylmethylether tropft man bei Raumtemperatur 40 ml Wasserstoffperoxid (35 prozentig) und erhitzt anschließend 4 h zum Sieden. Nach dem Abkühlen versetzt man mit 150 ml Wasser, trennt die Phasen und wäscht die organische Phase je zweimal mit Wasser und gesättigter Natriumsulfitlösung sowie mit gesättigter Natriumchloridlösung, so daß kein Peroxid mehr nachweisbar ist. Es wird mit Natriumsulfat getrocknet, und das Lösemittel wird am Rotationsverdampfer entfernt. Nach Umkristallisation aus Heptan erhält man 1,8-Difluorfluoren-2-ol.

Zu einer Mischung von 36 mmol 1,8-Difluorfluoren-2-ol und 109 mmol wasserfreiem Kaliumcarbonat in 250 ml 2-Butanon (Methylethylketon) gibt man 56 mmol 1-Brombutan und erhitzt anschließend 8 h unter Rückfluß. Die noch heiße Lösung wird mit 50 ml Wasser versetzt. Man trennt die Phasen, wäscht die organische Phase mit Wasser und gesättigter Natriumchloridlösung, trocknet mit Natriumsulfat und entfernt das Lösemittel am Rotationsverdampfer. Die Reinigung erfolgt durch Filtration über Aluminiumoxid (neutral) mit Toluol als Eluent und Umkristallisation aus Heptan. Man erhält 2-Butoxy-1,8-difluorfluoren.

Vergleichsbeispiel 2:

2-Butoxy-1,8-difluor-7-*n*-propylfluoren

Die Metallierung von 23 mmol 2-Butoxy-1,8-difluorfluoren mit 25 mmol *n*-Butyllithium (1,6 M Lösung in Hexan) und 25 mmol Kalium-*tert*-butylat erfolgt analog zum bei Vergleichsbeispiel 1 beschriebenen Vorgehen. Anschließend tropft man 28 mmol mmol Propionaldehyd bei ≤ -70°C zu und läßt langsam auftauen. Die Reaktionsmischung wird bei -10°C mit 50 ml Wasser versetzt und mit konzentrierter Salzsäure auf pH 1 angesäuert. Man extrahiert mit *tert*-Butylmethylether, wäscht die vereinigten organischen Phasen mit gesättigter Natriumchloridlösung, trocknet mit Natriumsulfat und entfernt die Lösemittel im Vakuum. Nach Chromatografie an Kieselgel mit Dichlormethan als Eluent erhält man 2-Butoxy-1,8-difluor-7-(1-hydroxypropyl)-fluoren.

Eine Lösung von 14 mmol 2-Butoxy-1,8-difluor-7-(1-hydroxypropyl)-fluoren in 50 ml Toluol wird nach Zusatz von ca. 0,5 g 4-Toluolsulfonsäure und einigen Tropfen konzentrierter Schwefelsäure 3 h am Wasserabscheider erhitzt. Nach dem Abkühlen extrahiert man mit 5 prozentiger Natriumhydrogencarbonatlösung und gesättigter Natriumchloridlösung und trocknet mit Natriumsulfat. Anschließend werden 0,5 g Palladium-Katalysator (10 prozentig auf Aktivkohle) zugegeben und bei Raumtemperatur hydriert. Die Reaktionsmischung wird filtriert, und das Lösemittel wird im Vakuum entfernt. Die Reinigung des Rohproduktes erfolgt durch Chromatografie an Kieselgel und Umkristallisation aus Heptan. Man erhält 2-Butoxy-1,8-difluor-7-*n*-propylfluoren.

Analog Vergleichsbeispiel 2, jedoch unter Einsatz von 4-*n*-Pentylcyclohexanon an Stelle von Propionaldehyd, kann erhalten werden:

Vergleichsbeispiel 3: 2-Butoxy-1,8-difluor-7-(*trans*-4-*n*-pentylcyclohexyl)fluoren

Vergleichsbeispiel 4:

1,8-Difluor-2-propylfluoren

Die Umsetzung von 1,8-Difluorfluoren mit Propionaldehyd erfolgt analog zu dem bei Vergleichsbeispiel 2 angegebenen Vorgehen. Man erhält 2-Propyl-1,8-difluorfluoren.

Analog Vergleichsbeispiel 2, jedoch unter Einsatz von 1,8-Difluor-2-propylfluoren (Vergleichsbeispiel 4), an Stelle von 2-Butoxy-1,8-difluorfluoren (Beispiel 1), kann erhalten werden:

Vergleichsbeispiel 5: 1,8-Difluor-2,7-dipropylfluoren

Analog Vergleichsbeispiel 1, jedoch unter Verwendung von 4,6-Difluordibenzofuran an Stelle von 1,8-Difluorfluoren und *n*-Butyllithium an Stelle der *n*-Butyllithium/ Kalium-*tert*-butylat-Mischung, erhält man:

Beispiel 1: 3-Butoxy-4,6-difluordibenzofuran

Ausgehend von 3-Butoxy-4,6-difluordibenzofuran (Beispiel 1) an Stelle von 2-Butyloxy-1,8-difluorfluoren (Vergleichsbeispiel 1), können analog Vergleichsbeispiel 2 und 3 erhalten werden:

Beispiel 2: 3-Butoxy-4,6-difluor-7-*n*-propyldibenzofuran

Beispiel 3: 3-Butoxy-4,6-difluor-7-(*trans*-4-*n*-pentylcyclohexyl)dibenzofuran

Analog Vergleichsbeispiel 4, jedoch unter Verwendung von 4,6-Difluordibenzofuran an Stelle von 1,8-Difluorfluoren und Valeraldehyd an Stelle von Propionaldehyd, kann erhalten werden:

Beispiel 4: 4,6-Difluor-3-*n*-pentyldibenzofuran

Analog Vergleichsbeispiel 5, jedoch unter Verwendung von 4,6-Difluor-3-*n*-pentyl-dibenzofuran (Beispiel 4) an Stelle von 1,8-Difluor-2-propylfluoren (Vergleichsbeispiel 4) und Valeraldehyd an Stelle von Propionaldehyd, kann erhalten werden:

Beispiel 5: 4,6-Difluor-3,7-di-*n*-pentyldibenzofuran

## Patentansprüche

1. Verbindung der Formel (I)

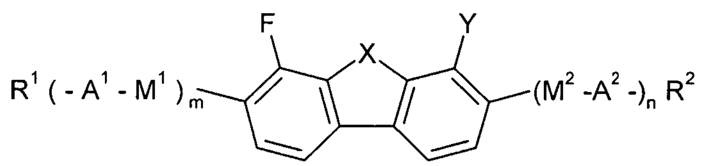

(I)

mit den Bedeutungen:

X ist C=O, O oder S,
Y ist H oder F,
$R^1$, $R^2$ sind gleich oder verschieden
Wasserstoff oder ein geradkettiger oder verzweigter Alkylrest mit 1 bis 16 C-Atomen oder ein geradkettiger oder verzweigter Alkenylrest mit 2 bis 16 C-Atomen, worin

a) eine oder mehrere nicht benachbarte und nicht terminale $CH_2$-Gruppen durch -O-, -C(=O)O-, -O-C(=O)-, -O-C(=O)-O-, -C(=O)- oder $-Si(CH_3)_2-$ ersetzt sein können, und/oder
b) eine $CH_2$-Gruppe durch -C≡C-, Cyclopropan-1,2-diyl oder Cyclobutan-1,3-diyl ersetzt sein kann, und/oder
c) ein oder mehrere H-Atome durch F oder Cl ersetzt sein können, und/oder
d) die terminale $CH_3$-Gruppe durch eine der folgenden chiralen Gruppen (optisch aktiv oder racemisch) ersetzt sein kann:

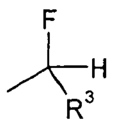 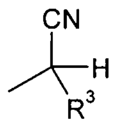 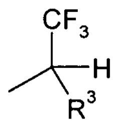 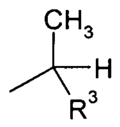

wobei $R^3$ ein geradkettiger oder verzweigter Alkylrest mit 1 bis 12 C-Atomen oder ein geradkettiger oder verzweigter Alkenylrest mit 2 bis 12 C-Atomen ist, worin eine oder mehrere nicht benachbarte und nicht terminale $CH_2$-Gruppen durch -O- ersetzt sein können und/oder ein oder mehrere H-Atome durch F oder Cl ersetzt sein können,

mit der Maßgabe, daß nur einer von $R^1$, $R^2$ Wasserstoff sein kann,
$M^1$, $M^2$ sind gleich oder verschieden -C(=O)-O-, -O-C(=O)-, $-CH_2-O-$, $-O-CH_2-$, $-CF_2-O-$, $-O-CF_2-$, -CH=CH-, -CF=CF-, -C≡C-, $-CH_2-CH_2-C(=O)-O-$, $-O-C(=O)-CH_2-CH_2-$, $-CH_2-CH_2-$, $-CF_2-CF_2-$, $-(CH_2)_4-$ oder eine Einfachbindung,
$A^1$, $A^2$ sind gleich oder verschieden
1,4-Phenylen, worin ein oder zwei H-Atome durch F, Cl oder CN ersetzt sein können, Pyridin-2,5-diyl, worin ein H-Atom durch F ersetzt sein kann, Pyrimidin-2,5-diyl, 1,4-Cyclohexylen, worin ein oder zwei H-Atome durch $CH_3$ oder F ersetzt sein können, 1-Cyclohexen-1,4-diyl, worin ein H-Atom durch $CH_3$ oder F ersetzt sein kann, oder 1,3-Dioxan-2,5-diyl,
m, n sind unabhängig voneinander 0 oder 1, in Summe jedoch nicht größer als 1.

2. Verbindung nach Anspruch 1, **dadurch gekennzeichnet, daß** in der Formel (I) bedeuten:

X gleich C=O oder O,
$R^1$, $R^2$ gleich oder verschieden
ein geradkettiger oder verzweigter Alkylrest mit 1, bis 14 C-Atomen oder ein geradkettiger oder verzweigter Alkenylrest mit 2 bis 14 C-Atomen, worin eine oder zwei nicht benachbarte und nicht terminale $CH_2$-Gruppen durch -O- ersetzt sein können und/oder ein oder mehrere H-Atome durch F ersetzt sein können,
$R^3$ ein geradkettiger oder verzweigter Alkylrest mit 1 bis 10 C-Atomen, worin eine nicht terminale $CH_2$-Gruppe durch -O- ersetzt sein kann,
$M^1$, $M^2$ gleich oder verschieden
$-CH_2-O-$, $-O-CH_2-$, $-CH_2-CH_2-$ oder eine Einfachbindung,
$A^1$, $A^2$ gleich oder verschieden
1,4-Phenylen, worin ein oder zwei H-Atome durch F ersetzt sein können, oder 1,4-Cyclohexylen,
m, n unabhängig voneinander 0 oder 1, in Summe jedoch nicht größer als 1.

3. Verbindung nach einem der Ansprüche 1 oder 2, **dadurch gekennzeichnet, daß** in der Formel (I)
X gleich O
bedeutet.

4. Verbindung nach einem oder mehreren der Ansprüche 1 bis 3, ausgewählt aus der Gruppe der Verbindungen der Formeln (I i) bis (I q)

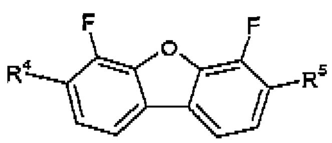

(I i)

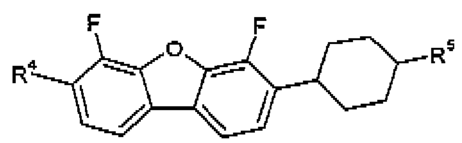

(I k)

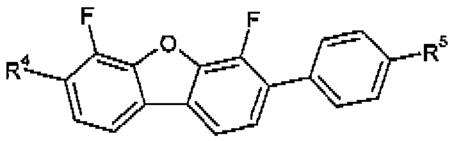

(I l)

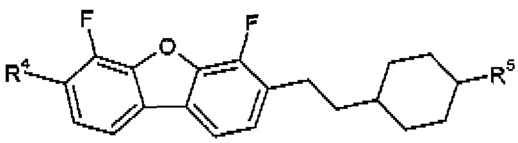

(I m)

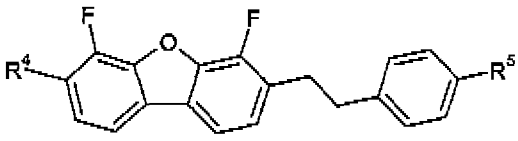

(I n)

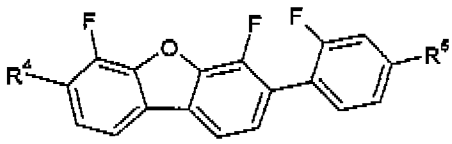

(I o)

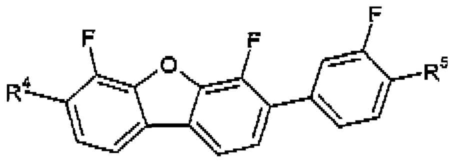

(I p)

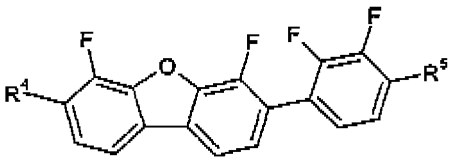

(I q)

worin bedeuten:

$R^4$ und $R^5$ gleich oder verschieden ein geradkettiger Alkyl-, Alkenyl- oder Alkoxyrest mit 2 bis 12 C-Atomen.

**5.** Verbindung nach Anspruch 4, ausgewählt aus der Gruppe der Verbindungen der Formeln (I i), (I k) und (Il), worin bedeuten:

$R^4$, $R^5$ gleich oder verschieden ein geradkettiger Alkyl-, Alkenyl- oder Alkoxyrest mit 2 bis 10 C-Atomen.

**6.** Verbindung der Formeln (I i) nach Anspruch 4 oder 5.

**7.** Verwendung von Verbindungen der Formel (I) gemäß einem der Ansprüche 1 bis 6 in Flüssigkristallmischungen.

**8.** Flüssigkristallmischung, enthaltend eine oder mehrere Verbindungen gemäß einem der Ansprüche 1 bis 6 in einer Menge von 1 bis 40 Gew.-%, bezogen auf die Flüssigkristallmischung.

**9.** Flüssigkristallmischung nach Anspruch 8, **dadurch gekennzeichnet, daß** sie nematisch ist.

**10.** Flüssigkristallmischung nach Anspruch 8, **dadurch gekennzeichnet, daß** sie smektisch ist.

**11.** Flüssigkristallmischung nach einem oder mehreren der Ansprüche 8 bis 10, enthaltend neben der/den Verbindung(en) der Formel (I) mindestens drei weitere Komponenten.

**12.** Flüssigkristalldisplay, enthaltend eine Flüssigkristallmischung gemäß einem oder mehreren der Ansprüche 8 bis 11.

**13.** Flüssigkristalldisplay nach Anspruch 12, **dadurch gekennzeichnet, daß** es Aktivmatrix-Elemente enthält.

**14.** Flüssigkristalldisplay nach Anspruch 12, **dadurch gekennzeichnet, daß** es im Inverse-Mode betrieben werden kann.

**15.** Flüssigkristalldisplay nach Anspruch 12 oder 13, **dadurch gekennzeichnet, daß** es im ECB- oder VA-Modus betrieben werden kann.

## Claims

**1.** Compound of the formula (I)

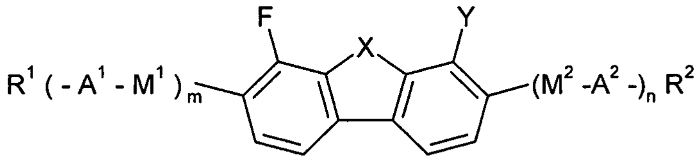

(I)

with the meanings:

X is C=O, O or S,
Yis HorF,
$R^1$, $R^2$ are, identically or differently,
hydrogen or a straight-chain or branched alkyl radical having 1 to 16 C atoms or a straight-chain or branched alkenyl radical having 2 to 16 C atoms, in which

a) one or more non-adjacent and non-terminal $CH_2$ groups may be replaced by -O-, -C(=O)O-, -O-C(=O)-, -O-C(=O)-O-, -C(=O)- or $-Si(CH_3)_2-$, and/or
b) one $CH_2$ group may be replaced by -C≡C-, cyclopropane-1,2-diyl or cyclobutane-1,3-diyl, and/or
c) one or more H atoms may be replaced by F or Cl, and/or
d) the terminal $CH_3$ group may be replaced by one of the following chiral groups (optically active or racemic):

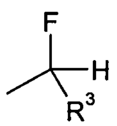
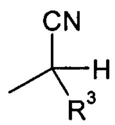
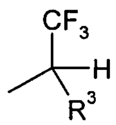
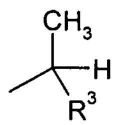

where $R^3$ is a straight-chain or branched alkyl radical having 1 to 12 C atoms or a straight-chain or branched alkenyl radical having 2 to 12 C atoms, in which one or more non-adjacent and non-terminal $CH_2$ groups may be replaced by-O- and/or one or more H atoms may be replaced by F or Cl,

with the proviso that only one of $R^1$, $R^2$ can be hydrogen,
$M^1$, $M^2$ are, identically or differently,
-C(=O)-O-, -O-C(=O)-, $-CH_2-O-$, $-O-CH_2-$, $-CF_2-O-$, $-O-CF_2-$, -CH=CH-, -CF=CF-, -C≡C-, $-CH_2-CH_2-C(=O)-O-$, $-O-C(=O)-CH_2-CH_2-$, $-CH_2-CH_2-$, $-CF_2-CF_2-$, $-(CH_2)_4-$ or a single bond,
$A^1$, $A^2$ are, identically or differently,
1,4-phenylene, in which one or two H atoms may be replaced by F, Cl or CN, pyridine-2,5-diyl, in which one H atom may be replaced by F, pyrimidine-2,5-diyl, 1,4-cyclohexylene, in which one or two H atoms may be replaced by $CH_3$ or F, 1-cyclohexene-1,4-diyl, in which one H atom may be replaced by $CH_3$ or F, or 1,3-dioxane-2,5-diyl,
m, n are, independently of one another, 0 or 1, but in total are not greater than 1.

**2.** Compound according to Claim 1, **characterised in that**, in the formula (I):

X is equal to C=O or O,
$R^1$, $R^2$ denote, identically or differently,
a straight-chain or branched alkyl radical having 1 to 14 C atoms or a straight-chain or branched alkenyl radical having 2 to 14 C atoms, in which one or two non-adjacent and non-terminal $CH_2$ groups may be replaced by -O- and/or one or more H atoms may be replaced by F,
$R^3$ denotes a straight-chain or branched alkyl radical having 1 to 10 C atoms, in which one non-terminal $CH_2$ group may be replaced by -O-,
$M^1$, $M^2$ denote, identically or differently,
$-CH_2-O-$, $-O-CH_2-$, $-CH_2-CH_2-$ or a single bond,
$A^1$, $A^2$ denote, identically or differently,

1,4-phenylene, in which one or two H atoms may be replaced by F, or 1,4-cyclohexylene,
m, n denote, independently of one another, 0 or 1, but in total are not greater than 1.

**3.** Compound according to one of Claims 1 or 2, **characterised in that**, in the formula (I), X is equal to O.

**4.** Compound according to one or more of Claims 1 to 3, selected from the group of the compounds of the formulae (I i) to (I q)

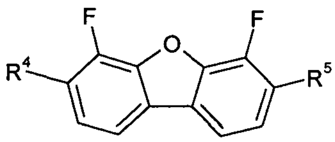

(I i)

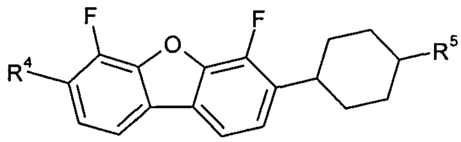

(I k)

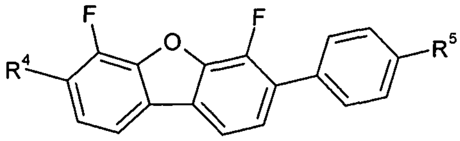

(I l)

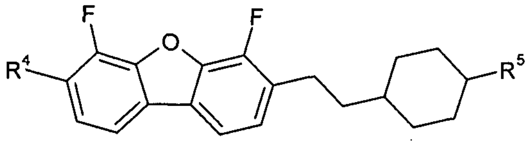

(I m)

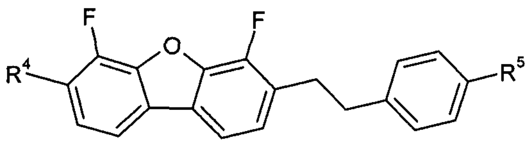

(I n)

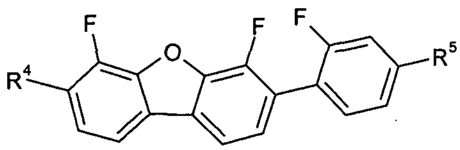

(I o)

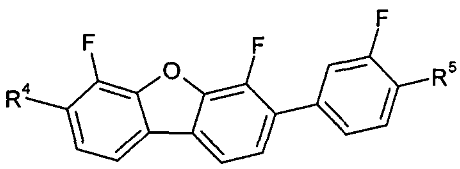

(I p)

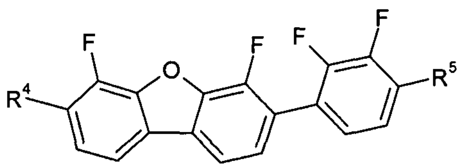

(I q)

in which:

$R^4$ and $R^5$ denote, identically or differently, a straight-chain alkyl, alkenyl or alkoxy radical having 2 to 12 C atoms.

**5.** Compound according to Claim 4, selected from the group of the compounds of the formulae (I i), (I k) and (I1) in which:

$R^4$, $R^5$ denote, identically or differently, a straight-chain alkyl, alkenyl or alkoxy radical having 2 to 10 C atoms.

**6.** Compound of the formula (I i) according to Claim 4 or 5.

**7.** Use of compounds of the formula (I) according to one of Claims 1 to 6 in liquid-crystal mixtures.

**8.** Liquid-crystal mixture comprising one or more compounds according to one of Claims 1 to 6 in an amount of 1 to 40% by weight, based on the liquid-crystal mixture.

**9.** Liquid-crystal mixture according to Claim 8, **characterised in that** it is nematic.

**10.** Liquid-crystal mixture according to Claim 8, **characterised in that** it is smectic.

**11.** Liquid-crystal mixture according to one or more of Claims 8 to 10, comprising, besides the compound(s) of the formula (I), at least three further components.

**12.** Liquid-crystal display containing a liquid-crystal mixture according to one or more of Claims 8 to 11.

**13.** Liquid-crystal display according to Claim 12, **characterised in that** it contains active-matrix elements.

**14.** Liquid-crystal display according to claim 12, **characterised in that** it can be operated in inverse mode.

**15.** Liquid-crystal display according to Claim 12 or 13, **characterised in that** it can be operated in ECB or VA mode.

**Revendications**

**1.** Composé de la formule (I) :

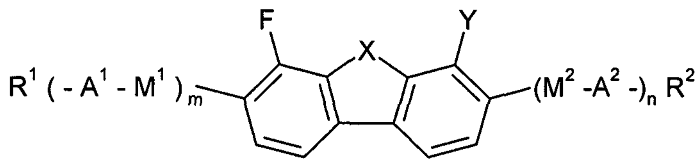

(I)

avec les significations :

X est C=O, O ou S,
Y est H ou F,
$R^1$, $R^2$ sont, de manière identique ou différente,
hydrogène ou un radical alkyle en chaîne droite ou ramifié comportant 1 à 16 atome(s) de C ou un radical alkényle en chaîne droite ou ramifié comportant 2 à 16 atomes de C, dans laquelle :

a) un ou plusieurs groupe(s) $CH_2$ non adjacents et non terminal/terminaux peut/peuvent être remplacé(s) par -O-, -C(=O)O-, -O-C(=O)-, -O-C(=O)-O-, -C(=O)- ou $-Si(CH_3)_2-$, et/ou
b) un groupe $CH_2$ peut être remplacé par -C≡C-, cyclopropane-1,2-diyle ou cyclobutane-1,3-diyle, et/ou
c) un ou plusieurs atome(s) de H peut/peuvent être remplacé(s) par F ou Cl, et/ou
d) le groupe $CH_3$ terminal peut être remplacé par l'un des groupes chiraux qui suivent (optiquement actifs ou racémiques) :

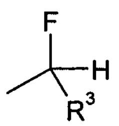 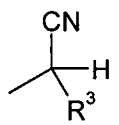 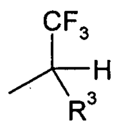 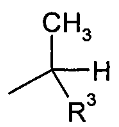

où $R^3$ est un radical alkyle en chaîne droite ou ramifié comportant 1 à 12 atome(s) de C ou un radical alkényle en chaîne droite ou ramifié comportant 2 à 12 atomes de C, où un ou plusieurs groupe(s) $CH_2$ non adjacents et non terminal/terminaux peut/peuvent être remplacé(s) par -O- et/ou un ou plusieurs atome(s) de H peut/peuvent être remplacé(s) par F ou Cl,

étant entendu que seulement l'un de $R^1$, $R^2$ peut être hydrogène,
$M^1$, $M^2$ sont, de manière identique ou différente,
-C(=O)-O-, -O-C(=O)-, $-CH_2-O-$, $-O-CH_2-$, $-CF_2-O-$, $-O-CF_2-$, -CH=CH-, -CF=CF-, -C≡C-, $-CH_2-CH_2-C(=O)-O-$, $-O-C(=O)-CH_2-CH_2-$, $-CH_2-CH_2-$, $-CF_2-CF_2-$, $-(CH_2)_4-$ ou une liaison simple,
$A^1$, $A^2$ sont, de manière identique ou différente,
1,4-phénylène, où un ou deux atome(s) de H peut/peuvent être remplacé(s) par F, Cl ou CN, pyridine-2,5-diyle,

où un atome de H peut être remplacé par F, pyrimidine-2,5-diyle, 1,4-cyclohexylène, où un ou deux atome(s) de H peut/peuvent être remplacé(s) par $CH_3$ ou F, 1-cyclohexène-1,4-diyle, où un atome de H peut être remplacé par $CH_3$ ou F, ou 1,3-dioxane-2,5-diyle,
m, n sont, indépendamment l'un de l'autre, 0 ou 1, mais leur total n'est pas supérieur à 1.

**2.** Composé selon la revendication 1, **caractérisé en ce que**, dans la formule (I):

X est égal à C=O ou O,
$R^1$, $R^2$ représentent, de manière identique ou différente,
un radical alkyle en chaîne droite ou ramifié comportant 1 à 14 atome(s) de C ou un radical alkényle en chaîne droite ou ramifié comportant 2 à 14 atomes de C, où un ou deux groupe(s) $CH_2$ non adjacents et non terminal/terminaux peut/peuvent être remplacé(s) par -O- et/ou un ou plusieurs atome(s) de H peut/peuvent être remplacé(s) par F,
$R^3$ représente un radical alkyle en chaîne droite ou ramifié comportant 1 à 10 atome(s) de C, où un groupe $CH_2$ non terminal peut être remplacé par -O-,
$M^1$, $M^2$ représentent, de manière identique ou différente,
$-CH_2-O-$, $-O-CH_2-$, $-CH_2-CH_2-$ ou une liaison simple,
$A^1$, $A^2$ représentent, de manière identique ou différente, 1,4-phénylène, où un ou deux atome(s) de H peut/peuvent être remplacé(s) par F, ou 1,4-cyclohexylène,
m, n représentent, indépendamment l'un de l'autre, 0 ou 1, mais leur total n'est pas supérieur à 1.

**3.** Composé selon l'une des revendications 1 ou 2, **caractérisé en ce que**, dans la formule (I),
X est égal à O.

**4.** Composé selon une ou plusieurs des revendications 1 à 3, choisi parmi le groupe des composés des formules (I i) à (I q) :

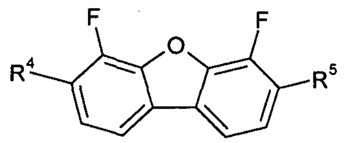

(I i)

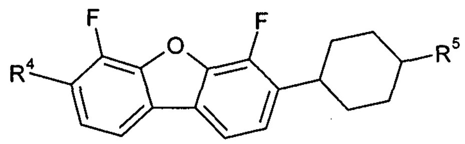

(I k)

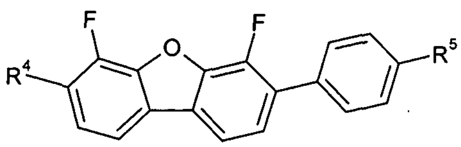

(I l)

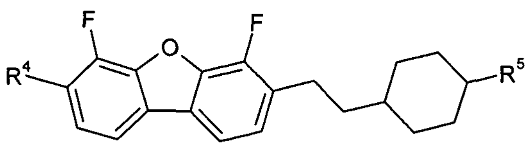

(I m)

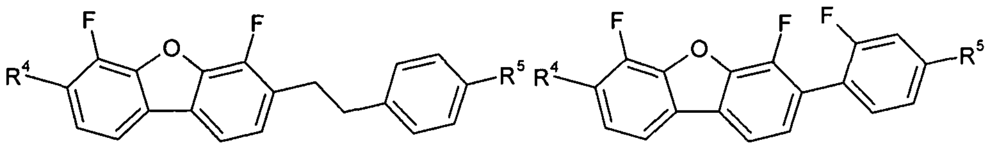

(I n) (I o)

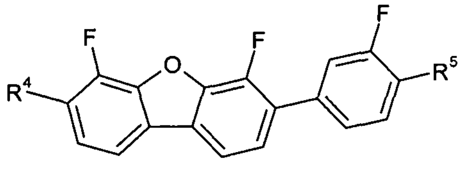

(I p)

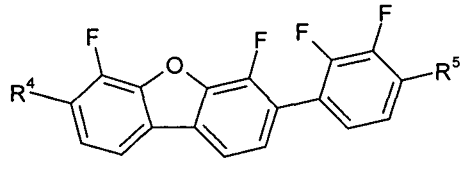

(I q)

dans lesquelles :

$R^4$ et $R^5$ représentent, de manière identique ou différente, un radical alkyle, alkényle ou alcoxy en chaîne droite comportant 2 à 12 atomes de C.

**5.** Composé selon la revendication 4, choisi parmi le groupe des composés des formules (I i), (Ik) et (I1) dans lesquelles :

$R^4$, $R^5$ représentent, de manière identique ou différente, un radical alkyle, alkényle ou alcoxy en chaîne droite comportant 2 à 10 atomes de C.

**6.** Composé de la formule (I i) selon la revendication 4 ou 5.

**7.** Utilisation de composés de la formule (I) selon l'une des revendications 1 à 6 dans des mélanges de cristaux liquides.

**8.** Mélange de cristaux liquides comprenant un ou plusieurs composé(s) selon l'une des revendications 1 à 6 selon une quantité de 1 à 40% en poids, sur la base du mélange de cristaux liquides.

**9.** Mélange de cristaux liquides selon la revendication 8, **caractérisé en ce qu'**il est nématique.

**10.** Mélange de cristaux liquides selon la revendication 8, **caractérisé en ce qu'**il est smectique.

**11.** Mélange de cristaux liquides selon une ou plusieurs des revendication 8 à 10, comprenant, en plus du/des composé(s) de la formule (I), au moins trois autres composants.

**12.** Affichage à cristaux liquides contenant un mélange de cristaux liquides selon une ou plusieurs des revendications 8 à 11.

**13.** Affichage à cristaux liquides selon la revendication 12, **caractérisé en ce qu'**il contient des éléments de matrice active.

**14.** Affichage à cristaux liquides selon la revendication 12, **caractérisé en ce qu'**il peut être actionné en mode inverse.

**15.** Affichage à cristaux liquides selon la revendication 12 ou 13, **caractérisé en ce qu'**il peut être actionné en mode ECB ou VA.

Fig.1

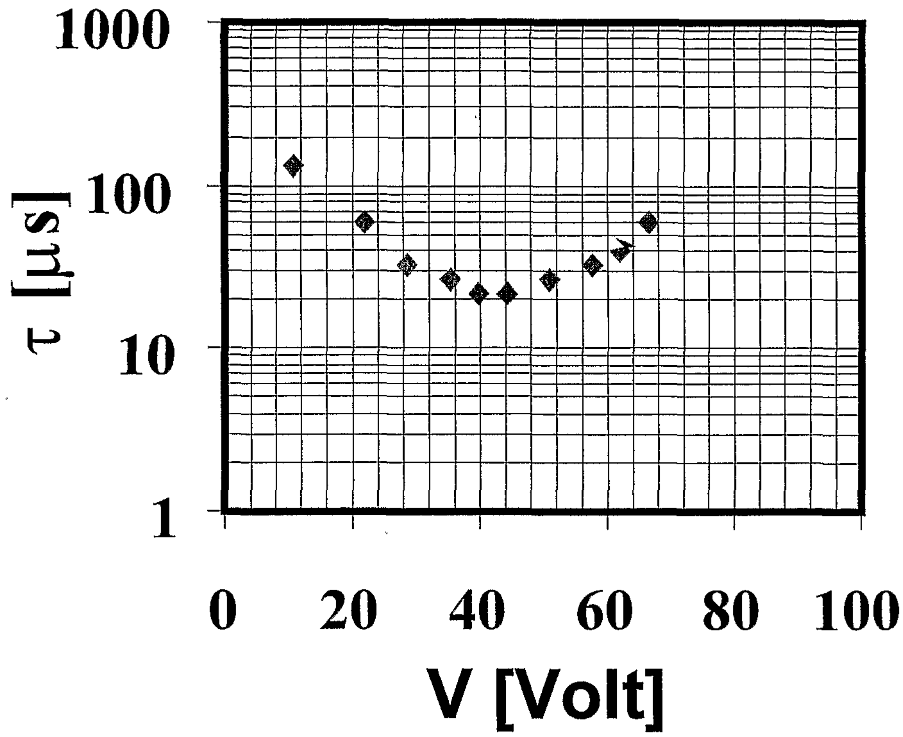

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4367924 A, Clark und Lagerwall
- EP 0032362 A
- WO 9712355 A
- WO 9960441 A
- DE 19720289 A
- EP 0113293 A
- EP 0325035 B
- JP 5125055 A
- JP 2000178211 A
- EP 0455219 A
- JP 10236992 A
- WO 0036054 A
- WO 9704039 A
- DE 19531165 A
- EP 0893424 A
- WO 9600710 A
- WO 9630344 A
- WO 9211241 A
- EP 0665825 A
- DE 4427266 A
- DE 19607996 A
- DE 19528665 A
- EP 0736513 A
- EP 0355008 A
- US 4798680 A
- EP 0552658 A
- EP 0318423 A


### In der Beschreibung aufgeführte Nicht-Patentliteratur


- **B. T. TSUKUDA.** TFT/LCD: Liquid Crystal Displays Addressed by Thin-Film Transistors. Gordon and Breach, 1996
- *Displays,* 1993, vol. 14 (2), 86-93
- *Kontakte,* 1993, 3-14
- *Ferroelectrics,* 1996, vol. 179, 141-152
- **B. T. MATSUMOTO et al.** *Proc. of the 6th Int. Display Research Conf.,* 30. September 1986, 468-470
- **M. MURAKAMI et al.** *PROC. OF THE 6TH INT. DISPLAY RESEARCH CONF.,* 344-347
- *Mol. Cryst. Liq. Cryst.,* 1983, vol. 94, 213
- *MOL. CRYST. LIQ. CRYST.,* 1984, vol. 114, 151
- **B. S. T. LAGERWALL et al.** Ferroelectric Liquid Crystals for Displays. *SID Symposium, Oct. Meeting,* 1985
- *J. Chem. Soc.,* 1955, 4359
- *J. CHEM. SOC.,* 1957, 3228
- *Pramana Suppl.,* 1975, vol. 1, 397
- *Bull. Soc. Chim. Fr.,* 1975, 2066
- *Mol. Cryst. Liq. Cryst,* 1985, vol. 127, 341
- *MOL. CRYST. LIQ. CRYST,* 1985, vol. 129, 17
- *MOL. CRYST. LIQ. CRYST,* 1987, vol. 150B, 361
- *MOL. CRYST. LIQ. CRYST,* 1988, vol. 164, 167
- *MOL. CRYST. LIQ. CRYST,* 1990, vol. 182, 339
- *Nippon Kagaku Kaishi,* 1989, vol. 12, 2052-2058
- *CAN,* 1990, vol. 112, 234935s
- Active Matrix Displays. AM-LCD) (siehe z. B. C. Prince, Seminar Lecture Notes. SID International Symposium, 1997, vol. I, M-3, , 3-M-22
- **BAHADUR.** Liquid Crystal Applications and Uses. World Scientific Publishing, 1990, vol. 1, 410
- **E. LÜDER.** *Recent Progress of AMLCD's, Proceedings of the 15th International Display Research Conference,* 1995, 9-12
- **J. C. JONES ; M. J. TOWLER ; J. R. HUGHES.** *Displays,* 1993, vol. 14 (2), 86-93
- **M. KODEN.** *Ferroelectrics,* 1996, vol. 179, 121-129
- **B. E. KANEKO.** Liquid Crystal TV Displays: Principles and Applications of Liquid Crystal Displays. KTK Scientific Publishers, 1987
- **B. J. C. JONES ; M. J. TOWLER ; J. R. HUGHES.** *Displays,* 1993, vol. 14 (2), 86-93
- *Nippon Kagaku Kaishi,* 1989, vol. 12, 2052
- *Yamaguchi Daigaku Kogakubu Kenkyu Hokoku,* 1995, vol. 45, 281
- *J. Org. Chem.,* 1968, vol. 33, 2575
- Arbeitsvorschrift: Organikum. VEB Deutscher Verlag der Wissenschaften, 1984
- *J. Am. Chem. Soc.,* 1974, vol. 96, 3536
- *Ref. Zh., Khim.,* 1983
- *Tetrahedron: Asymmetry,* 1999, vol. 10, 2417
- *J. Am. Chem. Soc.,* 1998, vol. 120, 3074
- *Tetrahedron,* 1997, vol. 53, 17425
- *J. Chem. Soc., Chem. Commun.,* 1995, vol. 15, 1515

- *Dibenzothiophen: Acta Pharm. Suec.,* 1978, vol. 15, 337
- *Synlett,* 1990, 747
- *SYNLETT,* 1991, 119
- *Tetrahedron Lett.,* 1996, vol. 37, 6551
- *Liq. Cryst.,* 1995, vol. 18, 1
- *Mol. Cryst. Liq. Cryst.,* 1991, vol. 204, 43
- *Liq. Cryst.,* 1997, vol. 23, 389
- *Synthesis,* 1996, 589
- *J. Mater. Chem.,* 1999, vol. 9, 1669
- *J. Org. Chem.,* 1984, vol. 49, 2534-2540
- *Bull. Korean. Chem. Soc.,* 1999, vol. 20, 1373
- *Tetrahedron Lett.,* 1968, vol. 30, 3363
- *Liq. Cryst.,* 1997, vol. 23, 69
- *J. Org. Chem.,* 1981, vol. 46, 3917
- *J. Chem. Soc., Chem. Commun.,* 1995, 177
- *Synlett,* 1991, 191
- *Tetrahedron,* 1996, vol. 52, 9